# EUROPEAN PATENT SPECIFICATION

(11) **EP 2 213 296 B1**
(45) Date of publication and mention of the grant of the patent: **15.04.2015**
(21) Application number: 08837820.3
(22) Date of filing: 10.10.2008
(51) Int. Cl.: A61K 38/18, A61P 17/04, A61P 39/00, C12N 15/62

(54) **FGF1/FGF2 CHIMERIC PROTEIN AND USES THEREOF**
FGF1/FGF CHIMÄRES PROTEIN UND VERWENDUNG
PROTEINE CHIMÈRE FGF1/FGF2 ET SON UTILISATION

(30) Priority: 12.10.2007 JP 2007267000; 23.10.2007 JP 2007275496; 16.07.2008 JP 2008184952
(43) Date of publication of application: 04.08.2010
(73) Proprietor: National Institute of Advanced Industrial Science and Technology, Tokyo 100-8921 (JP)
(72) Inventor: IMAMURA, Toru, Tsukuba-shi Ibaraki 305-8566 (JP); MOTOMURA, Kaori, Tsukuba-shi Ibaraki 305-8566 (JP); KURAMOCHI, Akiko, Tsukuba-shi Ibaraki 305-8566 (JP); HANYU, Yoshiro, Tsukuba-shi Ibaraki 305-8566 (JP); SUZUKI, Masashi, Tsukuba-shi Ibaraki 305-8566 (JP); ASADA, Masahiro, Tsukuba-shi Ibaraki 305-8566 (JP); HAGIWARA, Akiko, Funabashi-shi, Chiba 273-0044 (JP); NAKAYAMA, Fumiaki, Chiba 263-8555 (JP); AKASHI, Makoto, Tsukuba-shi Ibaraki 305-8566 (JP)
(74) Representative: Tuxworth, Pamela M.
(86) International application number: PCT/JP2008/068413
(87) International publication number: WO 2009/048119

(56) References cited:
- EP-A1- 1 444 995
- WO-A1-92/11360
- WO-A1-99/01150
- JP-A- 8 310 966
- US-A1- 2005 227 329
- MOTOMURA K ET AL: "An FGF1:FGF2 chimeric growth factor exhibits universal FGF receptor specificity, enhanced stability and augmented activity useful for epithelial proliferation and radioprotection", BIOCHIMICA ET BIOPHYSICA ACTA - GENERAL SUBJECTS, ELSEVIER SCIENCE PUBLISHERS, NL, vol. 1780, no. 12, 12 August 2008 (2008-08-12), pages 1432-1440, XP025470931, ISSN: 0304-4165, DOI: 10.1016/J.BBAGEN.2008.08.001 [retrieved on 2008-08-12]
- NAKAYAMA F ET AL: "Post Treatment With an FGF Chimeric Growth Factor Enhances Epithelial Cell Proliferation to Improve Recovery From Radiation-Induced Intestinal Damage", INTERNATIONAL JOURNAL OF RADIATION: ONCOLOGY BIOLOGY PHYSICS, PERGAMON PRESS, USA, vol. 78, no. 3, 1 November 2010 (2010-11-01), pages 860-867, XP027325940, ISSN: 0360-3016 [retrieved on 2010-08-21]
- IMAMURA TORU ET AL: "A novel chimeric fibroblast growth factor for liver parenchymal cells", HEPATOLOGY, WILLIAMS AND WILKINS, BALTIMORE, MD, US, vol. 23, no. 2, 1 January 1996 (1996-01-01), pages 316-319, XP009159017, ISSN: 0270-9139, DOI: 10.1002/HEP.510230218 [retrieved on 2003-12-30]
- PENG HU ET AL: "Structural Basis for Differences in Heparin-Dependence of Endothelial Cell Proliferation Activities of FGF-1 and FGF-2", BLOOD, AMERICAN SOCIETY OF HEMATOLOGY, US, vol. 100, no. 11, 16 November 2002 (2002-11-16), page 72B, XP009159066, ISSN: 0006-4971
- PENG HU ET AL: "Identification of the binding site for human fibrinogen for FGF-2", BLOOD, AMERICAN SOCIETY OF HEMATOLOGY, US, vol. 102, no. 11, 16 November 2003 (2003-11-16), page 782A, XP009159065, ISSN: 0006-4971
- IMAMURA,T. ET AL.: 'Identification of the domain within fibroblast growth factor-1 responsible for heparin-dependence' BIOCHIM BIOPHYS ACTA vol. 1266, no. 2, 1995, pages 124 - 30, XP000930022
- 'Proceedings of the Annual Meeting of the Japan Radiation Research Society', vol. 49, 2006 article AKIKO OGIWARA ET AL.: 'FGF1 no Hoshasen Shocho Shogai ni Taisuru Bogo Koka ni Tsuite', page 155, XP008138479
- OKUNIEFF,P. ET AL.: 'In vivo radioprotective effects of angiogenic growth factors on the small bowel of C3H mice' RADIAT RES vol. 150, no. 2, 1998, pages 204 - 11, XP008134721
- EVARTS,R.P. ET AL.: 'Activation of hepatic stem cell compartment in the rat: role of transforming growth factor alpha, hepatocyte growth factor, and acidic fibroblast growth factor in early proliferation' CELL GROWTH DIFFER vol. 4, no. 7, 1993, pages 555 - 61, XP008134414
- BOS,C. ET AL.: 'Human mesenchymal stem cells respond to fibroblast growth factors' HUM CELL vol. 10, no. 1, 1997, pages 45 - 50, XP009060967

### SEQUENCE LISTING

<110> National Institute of Advanced Industrial Science and Technology (
AIST)
<120> FGF1/FGF2 kimera medicines
<130> 2008002922
<150> JP2007/267000
   <151> 2007-10-12
<150> JP2007/275496
   <151> 2007-10-24
<150> JP2008/184952
   <151> 2008-07-16
<160> 10
<170> Patent inversion 3.1
<210> 1
   <211> 155
   <212> PRT
   <213> Artificial
<220>
   <223> FGFC1
<220>
   <221> MISC_FEATURE
   <222> (83).. (83)
   <223> Xaa=Asp, Lys or Glu
<400> 1
   Met Ala Glu Gly Glu Ile Thr Thr Phe Thr Ala Leu Thr Glu Lys Phe
   1               5                   10                  15
   Asn Leu Pro Pro Gly Asn Tyr Lys Lys Pro Lys Leu Leu Tyr Cys Ser
               20                  25                  30
   Asn Gly Gly His Phe Leu Arg Ile His Pro Asp Gly Arg Val Asp Gly
           35                  40                  45
   Val Arg Glu Lys Ser Asp Pro His Ile Lys Leu Gln Leu Gln Ala Glu
   50 55 60
   Glu Arg Gly Val Val Ser Ile Lys Gly Val Cys Ala Asn Arg Tyr Leu
   65 70 75 80
   Ala Met Xaa Thr Asp Gly Leu Leu Tyr Gly Ser Gln Thr Pro Asn Glu
   85 90 95
   Glu Cys Leu Phe Leu Glu Arg Leu Glu Glu Asn His Tyr Asn Thr Tyr
   100 105 110
   Ile Ser Lys Lys His Ala Glu Lys Asn Trp Phe Val Gly Leu Lys Lys
   115 120 125
   Asn Gly Ser Cys Lys Arg Gly Pro Arg Thr His Tyr Gly Gln Lys Ala
   130 135 140
   Ile Leu Phe Leu Pro Leu Pro Val Ser Ser Asp
   145 150 155
<210> 2
   <211> 136
   <212> PRT
   <213> Artificial
<220>
   <223> FGFC1 (-21aa)
<220>
   <221> MISC_FEATURE
   <222> (64).. (64)
   <223> Xaa=Asp, Lys orGlu
<400> 2
   Met Ala Asn Tyr Lys Lys Pro Lys Leu Leu Tyr Cys Ser Asn Gly Gly
   1 5 10 15
   His Phe Leu Arg Ile His Pro Asp Gly Arg Val Asp Gly Val Arg Glu
   20 25 30
   Lys Ser Asp Pro His Ile Lys Leu Gln Leu Gln Ala Glu Glu Arg Gly
   35 40 45
   Val Val Ser Ile Lys Gly Val Cys Ala Asn Arg Tyr Leu Ala Met Xaa
   50 55 60
   Thr Asp Gly Leu Leu Tyr Gly Ser Gln Thr Pro Asn Glu Glu Cys Leu
   65 70 75 80
   Phe Leu Glu Arg Leu Glu Glu Asn His Tyr Asn Thr Tyr Ile Ser Lys
   85 90 95
   Lys His Ala Glu Lys Asn Trp Phe Val Gly Leu Lys Lys Asn Gly Ser
   100 105 110
   Cys Lys Arg Gly Pro Arg Thr His Tyr Gly Gln Lys Ala Ile Leu Phe
   115 120 125
   Leu Pro Leu Pro Val Ser Ser Asp
   130 135
<210> 3
   <211> 155
   <212> PRT
   <213> Artificial
<220>
   <223> FGFC2
<220>
   <221> MISC_FEATURE
   <222> (83).. (83)
   <223> Xaa=Asp, Lys orGlu
<400> 3
   Met Ala Glu Gly Glu Ile Thr Thr Phe Thr Ala Leu Thr Glu Lys Phe
   1 5 10 15
   Asn Leu Pro Pro Gly Asn Tyr Lys Lys Pro Lys Leu Leu Tyr Cys Ser
   20 25 30
   Asn Gly Gly His Phe Leu Arg Ile Leu Pro Asp Gly Thr Val Asp Gly
   35 40 45
   Thr Arg Asp Arg Ser Asp Gln His Ile Gln Leu Gln Leu Gln Ala Glu
   50 55 60
   Glu Arg Gly Val Val Ser Ile Lys Gly Val Cys Ala Asn Arg Tyr Leu
   65 70 75 80
   Ala Met Xaa Thr Asp Gly Leu Leu Tyr Gly Ser Gln Thr Pro Asn Glu
   85 90 95
   Glu Cys Leu Phe Leu Glu Arg Leu Glu Glu Asn His Tyr Asn Thr Tyr
   100 105 110
   Ile Ser Lys Lys His Ala Glu Lys Asn Trp Phe Val Gly Leu Lys Lys
   115 120 125
   Asn Gly Ser Cys Lys Arg Gly Pro Arg Thr His Tyr Gly Gln Lys Ala
   130 135 140
   Ile Leu Phe Leu Pro Leu Pro Val Ser Ser Asp
   145 150 155
<210> 4
   <211> 136
   <212> PRT
   <213> Artificial
<220>
   <223> FGFC2(-21aa)
<220>
   <221> MISC_FEATURE
   <222> (64).. (64)
   <223> Xaa=Asp, Lys orGlu
<400> 4
   Met Ala Asn Tyr Lys Lys Pro Lys Leu Leu Tyr Cys Ser Asn Gly Gly
   1               5                   10                  15
   His Phe Leu Arg Ile Leu Pro Asp Gly Thr Val Asp Gly Thr Arg Asp
               20                  25                  30
   Arg Ser Asp Gln His Ile Gln Leu Gln Leu Gln Ala Glu Glu Arg Gly
           35                  40                  45
   Val Val Ser Ile Lys Gly Val Cys Ala Asn Arg Tyr Leu Ala Met Xaa
       50                  55                  60
   Thr Asp Gly Leu Leu Tyr Gly Ser Gln Thr Pro Asn Glu Glu Cys Leu
   65                  70                  75                  80
   Phe Leu Glu Arg Leu Glu Glu Asn His Tyr Asn Thr Tyr Ile Ser Lys
                   85                  90                  95
   Lys His Ala Glu Lys Asn Trp Phe Val Gly Leu Lys Lys Asn Gly Ser
               100                 105                 110
   Cys Lys Arg Gly Pro Arg Thr His Tyr Gly Gln Lys Ala Ile Leu Phe
           115                 120                 125
   Leu Pro Leu Pro Val Ser Ser Asp
       130                 135
<210> 5
   <211> 136
   <212> PRT
   <213> Artificial
<220>
   <223> FGFC(MA/41-78/83D)
<400> 5
   Met Ala Asn Tyr Lys Lys Pro Lys Leu Leu Tyr Cys Ser Asn Gly Gly
   1 5 10 15
   His Phe Leu Arg Ile His Pro Asp Gly Arg Val Asp Gly Val Arg Glu
   20 25 30
   Lys Ser Asp Pro His Ile Lys Leu Gln Leu Gln Ala Glu Glu Arg Gly
   35 40 45
   Val Val Ser Ile Lys Gly Val Cys Ala Asn Arg Tyr Leu Ala Met Asp
   50 55 60
   Thr Asp Gly Leu Leu Tyr Gly Ser Gln Thr Pro Asn Glu Glu Cys Leu
   65 70 75 80
   Phe Leu Glu Arg Leu Glu Glu Asn His Tyr Asn Thr Tyr Ile Ser Lys
   85 90 95
   Lys His Ala Glu Lys Asn Trp Phe Val Gly Leu Lys Lys Asn Gly Ser
   100 105 110
   Cys Lys Arg Gly Pro Arg Thr His Tyr Gly Gln Lys Ala Ile Leu Phe
   115 120 125
   Leu Pro Leu Pro Val Ser Ser Asp
   130 135
<210> 6
   <211> 136
   <212> PRT
   <213> Artificial
<220>
   <223> FGFC(MA/62-78/83D)
<400> 6
   Met Ala Asn Tyr Lys Lys Pro Lys Leu Leu Tyr Cys Ser Asn Gly Gly
   1               5                   10                  15
   His Phe Leu Arg Ile Leu Pro Asp Gly Thr Val Asp Gly Thr Arg Asp
               20                  25                  30
   Arg Ser Asp Gln His Ile Gln Leu Gln Leu Gln Ala Glu Glu Arg Gly
           35                  40                  45
   Val Val Ser Ile Lys Gly Val Cys Ala Asn Arg Tyr Leu Ala Met Asp
       50                  55                  60
   Thr Asp Gly Leu Leu Tyr Gly Ser Gln Thr Pro Asn Glu Glu Cys Leu
   65                  70                  75                  80
   Phe Leu Glu Arg Leu Glu Glu Asn His Tyr Asn Thr Tyr Ile Ser Lys
                   85                  90                  95
   Lys His Ala Glu Lys Asn Trp Phe Val Gly Leu Lys Lys Asn Gly Ser
               100                 105                 110
   Cys Lys Arg Gly Pro Arg Thr His Tyr Gly Gln Lys Ala Ile Leu Phe
           115                 120                 125
   Leu Pro Leu Pro Val Ser Ser Asp
       130                 135
<210> 7
   <211> 135
   <212> PRT
   <213> Artificial
<220>
   <223> FGFC(M/41-78/83D)
<400> 7
   Met Asn Tyr Lys Lys Pro Lys Leu Leu Tyr Cys Ser Asn Gly Gly His
   1 5 10 15
   Phe Leu Arg Ile His Pro Asp Gly Arg Val Asp Gly Val Arg Glu Lys
   20 25 30
   Ser Asp Pro His Ile Lys Leu Gln Leu Gln Ala Glu Glu Arg Gly Val
   35 40 45
   Val Ser Ile Lys Gly Val Cys Ala Asn Arg Tyr Leu Ala Met Asp Thr
   50 55 60
   Asp Gly Leu Leu Tyr Gly Ser Gln Thr Pro Asn Glu Glu Cys Leu Phe
   65 70 75 80
   Leu Glu Arg Leu Glu Glu Asn His Tyr Asn Thr Tyr Ile Ser Lys Lys
   85 90 95
   His Ala Glu Lys Asn Trp Phe Val Gly Leu Lys Lys Asn Gly Ser Cys
   100 105 110
   Lys Arg Gly Pro Arg Thr His Tyr Gly Gln Lys Ala Ile Leu Phe Leu
   115 120 125
   Pro Leu Pro Val Ser Ser Asp
   130 135
<210> 8
   <211> 135
   <212> PRT
   <213> Artificial
<220>
   <223> FGFC(M/62-78/83D)
<400> 8
   Met Asn Tyr Lys Lys Pro Lys Leu Leu Tyr Cys Ser Asn Gly Gly His
   1 5 10 15
   Phe Leu Arg Ile Leu Pro Asp Gly Thr Val Asp Gly Thr Arg Asp Arg
   20 25 30
   Ser Asp Gln His Ile Gln Leu Gln Leu Gln Ala Glu Glu Arg Gly Val
   35 40 45
   Val Ser Ile Lys Gly Val Cys Ala Asn Arg Tyr Leu Ala Met Asp Thr
   50 55 60
   Asp Gly Leu Leu Tyr Gly Ser Gln Thr Pro Asn Glu Glu Cys Leu Phe
   65 70 75 80
   Leu Glu Arg Leu Glu Glu Asn His Tyr Asn Thr Tyr Ile Ser Lys Lys
   85 90 95
   His Ala Glu Lys Asn Trp Phe Val Gly Leu Lys Lys Asn Gly Ser Cys
   100 105 110
   Lys Arg Gly Pro Arg Thr His Tyr Gly Gln Lys Ala Ile Leu Phe Leu
   115 120 125
   Pro Leu Pro Val Ser Ser Asp
   130 135
<210> 9
   <211> 155
   <212> PRT
   <213> human FGF1
<400> 9
   Met Ala Glu Gly Glu Ile Thr Thr Phe Thr Ala Leu Thr Glu Lys Phe
   1 5 10 15
   Asn Leu Pro Pro Gly Asn Tyr Lys Lys Pro Lys Leu Leu Tyr Cys Ser
   20 25 30
   Asn Gly Gly His Phe Leu Arg Ile Leu Pro Asp Gly Thr Val Asp Gly
   35 40 45
   Thr Arg Asp Arg Ser Asp Gln His Ile Gln Leu Gln Leu Ser Ala Glu
   50 55 60
   Ser Val Gly Glu Val Tyr Ile Lys Ser Thr Glu Thr Gly Gln Tyr Leu
   65 70 75 80
   Ala Met Asp Thr Asp Gly Leu Leu Tyr Gly Ser Gln Thr Pro Asn Glu
   85 90 95
   Glu Cys Leu Phe Leu Glu Arg Leu Glu Glu Asn His Tyr Asn Thr Tyr
   100 105 110
   Ile Ser Lys Lys His Ala Glu Lys Asn Trp Phe Val Gly Leu Lys Lys
   115 120 125
   Asn Gly Ser Cys Lys Arg Gly Pro Arg Thr His Tyr Gly Gln Lys Ala
   130 135 140
   Ile Leu Phe Leu Pro Leu Pro Val Ser Ser Asp
   145 150 155
<210> 10
   <211> 155
   <212> PRT
   <213> human FGF2(Methionine-initiated translation product)
<400> 10

## Description

### Technical Field

The present invention relates to an FGF2 substitute-containing medicinal composition, which comprises, as an active ingredient, a chimeric protein, wherein a specific region of an acidic fibroblast growth factor (hereinafter referred to as "FGF1") protein is substituted with the corresponding region of a basic fibroblast growth factor (hereinafter referred to as "FGF2") protein. The present invention particularly relates to a medicinal composition effective for the promotion of wound healing, the prevention and treatment of radiation-induced damage to the intestinal canal, and the prevention and treatment of radiation-induced damage to the bone marrow. It also relates to an *in vitro* method of promotion of the proliferation of stem cells using such a medicinal composition.

### Background Art

As with FGF1, FGF2 is a fibroblast growth factor belonging to the FGF family. FGF2 shares many properties with FGF1. For example, FGF2 has the property of causing proliferation or migration of many cells as FGF1 does. FGF1 is able to exhibit its full biological activity only in the presence of heparin. In contrast, FGF2 which has the advantage of not being heparin-dependent has been widely used as an FGF2 medicinal composition.

In particular, an FGF2 medicinal composition used for wound healing completely differs from the conventionally used "povidone-iodine sucrose," which simply disinfects an affected area to prevent bacterial infection and waits for spontaneous skin regeneration. The FGF2 medicinal composition has been placed on the market as a revolutionary therapeutic agent for promoting wound healing by positively allowing skin cells to proliferate. This medicinal composition has been widely used in clinical settings, and it has already been on the market with the common name "Trafermin" and the product name "Fiblast Spray." The FGF2 medicinal composition is also considered to be effective as a fracture treating agent (Non-Patent Document 5), a periodontal disease treating agent (Non-Patent Document 6), an agent for preventing radiation-induced damage to the bone marrow (Non-Patent Document 7), an agent for preventing radiation-induced damage to the intestinal canal (Non-Patent Document 8), an agent for proliferating stem cells (Non-Patent Document 9), etc.

In Japan, a therapeutic agent containing FGF2 has been generally used as a type of therapeutic agent for promoting wound healing that heals the wound by allowing skin cells to proliferate. An FGF7 growth factor, which has recently been placed on the market in the U.S.A., is considered to be theoretically effective for the treatment of the epidermis from the viewpoint of receptor binding specificity. However, it is not able to promote the proliferation of dermal cells. Thus, when such FGF7 growth factor is used for the treatment of the skin, it is not considered suitable for the treatment of severe wounds such as ulcer and bedsore that even affect the dermis. As a result, the FGF7 growth factor is not indicated for wounds on the skin but it is officially indicated for the inflammation of the lining mucous membrane in the mouth caused by chemoradiation therapy.

On the other hand, since FGF2 is non-heparin-dependent and is able to promote the proliferation of dermal cells, it can be widely used in general wound healing including severe cases. However, because of its receptor binding specificity, FGF2 has extremely low reactivity to epithelial cells. Thus, although FGF2 is effective for the treatment of the dermis, it has been unable to directly promote the proliferation of epidermal cells. That is to say, while promoting the proliferation and regeneration of the dermis, FGF2 waits for the self repairing capability of epidermal cells to develop, eventually achieving the regeneration of the skin as a whole. Accordingly, FGF2 has the problem of requiring a long healing time. In particular, in the treatment of serious bedsore of elder people whose ability to regenerate the skin is significantly decreased, or the skin ulcer of patients suffering from diabetes, there have been many cases in which the disease is hardly treated. Thus, such FGF2 has not yet been satisfactory as an agent for treating the skin.

Moreover, an FGF2 protein is easily decomposed by protease existing in living bodies, and its activity is unstable in a temperature range between approximately 20°C and 40°C, which corresponds to the range from room temperature to a temperature close to body temperature. Thus, the FGF2 protein has been problematic in that its activity is rapidly lost when it is administered to a living body. Further, since such FGF2 protein is easily adsorbed on the wall of a storage vessel and it is also likely to form an aggregate, it easily disappears from a solution. Accordingly, it is difficult to maintain the activity of the protein after it has been processed into a formulation. Hence, it has also been desired to improve the FGF2 protein for use as a medicinal preparation.

Under such circumstances, it has been desired to develop an FGF2 substitute-containing medicinal composition that can be used as a skin wound healing promoter having activity of promoting the proliferation of epithelial cells such as epidermal cells while maintaining the advantages of FGF2 such as dermal cell proliferating activity and non-heparin-dependence and which is easy to formulate and exhibits high stability.

Patent Document 1: Japanese Patent No. 2733207

Non-Patent Document 1:Imamura T, Friedman SA, Gamble S, Tokita Y, Opalenik SR, Thompson JA, Maciag T. Identification of the domain within fibroblast growth factor-1 responsible for heparin-dependence. Biochim Biophys Acta. 1995 Apr 28;1266(2):124-30.

Non-Patent Document 2: T Imamura, T Tanahashi, A novel chimeric firbroblast growth facter for liver parenchymal cells Hepatology 1996 Volume 23, Issue 2, Pages 316-319

Non-Patent Document 3: Ornitz DM, Xu J, Colvin JS, McEwen DG, MacArthur CA, Coulier F, Gao G, Goldfarb M. Receptor specificity of the fibroblast growth factor family. J.Biol.Chem.1996 Jun 21;271(25) :15292-7.

Non-Patent Document 4: V.P. Eswarakumar, I. Lax, J. Schlessinger (2005) Cellular singnaling by fibroblast growth factor receptors. Cytokine & Growth Factor Reviews 16, 139-149

Non-Patent Document 5: Kawaguchi H, Nakamura K, Tabata Y, Ikada Y, Aoyama I, Anzai J, Nakamura T, Hiyama Y, Tamura M. Acceleration of Fracture Healing in Nonhuman Primates by Fibroblast Growth Factor-2. J Clinical Endocrinol Metabolism 86, 875-880, 2001

Non-Patent Document 6: Murakami S, Takayama S, Kitamura M, Shimabukuro Y, Yanagi K, Ikezawa K, Saho T, Nozaki T, Okada H. Recombinant human basic fibroblast growth factor (bFGF) stimulates periodontal regeneration in class II furcation defects created in beagle dogs. J Periodont Res. 38, 97-103, 2003

Non-Patent Document 7: Ding I, Huang K, Wang X, Greig JR, Miller RW, Okunieff P. Radioprotection of hematopoietic tissue by fibroblast growth factors in fractionated radiation experiments. Acta Oncol. 1997;36(3):337-340.

Non-Patent Document 8: Okunieff P, Mester M, Wang J, Maddox T, Gong X, Tang D, Coffee M, Ding I. In vivo radioprotective effects of angiogenic growth factors on the small bowel of C3H mice. Radiat Res. 150,204-211, 1998.

Non-Patent Document 9: Glaser T, Pollard SM, Smith A, Bruestle O. Tripotential differentiation of adherently expandable neural stem (NS) cells. PLoS ONE. 2007 Mar 14;2(3):e298.

Non-Patent Document 10: Forough R, Engleka K, Thompson JA, Jackson A, Imamura T, Maciag T. Differential expression in Escherichia coli of the alpha and beta forms of heparin-binding acidic fibroblast growth factor-1: potential role of RNA secondary structure. Biochim Biophys Acta. 1991 Nov 11;1090(3):293-8.

### Disclosure of the Invention

### Problems to be Solved by the Invention

It is an object of the present invention to provide an FGF2 substitute-containing medicinal composition, specifically, a medicinal composition effective for wound healing and the prevention and treatment of radiation-induced damage, which maintain the properties of FGF2, such as fibroblast proliferating activity, stem cell proliferation promoting activity, and non-heparin-dependence, and which also have an activity of promoting the proliferation of epithelial cells. The invention also provides an *in vitro* method of promoting the proliferation of stem cells using such a medicinal composition.

### Means for Solving the Problems

As FGF proteins that also have an activity of promoting the proliferation of epithelial cells such as epidermal cells, FGF1 having a wide range of receptor characteristics, is known in addition to FGF2. FGF1 acts on both dermal cells and epidermal cells and promotes the proliferation of such cells. However, its complete activity cannot be obtained without the addition of exogenous heparin. In the case of a wound or the inflammation of the intestinal canal, the affected area often becomes hemorrhagic. If the activity of antithrombin III is inhibited by heparin, blood coagulation is suppressed, thereby causing a great problem. Thus, FGF1, which essentially requires exogenous heparin, is not suitable as a therapeutic agent for wounds or the inflammation of the intestinal canal. Accordingly, as stated above, there has conventionally been no alternative but to use FGF2 to treat wounds on the skin although it has the disadvantage of not having an epithelial cell proliferating property. Furthermore, FGF1 has all of the aforementioned disadvantages of FGF2, such as easy degradability by protease, instability in a temperature range between approximately 20°C and 40°C, adsorption onto the wall of a vessel, and the formation of an aggregate. Still further, such disadvantages of FGF1 are more noticeable than those of FGF2. Thus, FGF1 has not been attractive as a target for development of an alternative useful medicament.

In past days (in 1995), the present inventors were producing a large number of chimeric proteins in which a specific region of an FGF1 protein was substituted with the corresponding region of an FGF2 protein (Non-Patent Document 1), and they were examining the properties of those chimeric proteins. In the process, the inventors found that a chimeric protein (which is referred to as "FGF-C(1211) in Non-Patent Document 1 and hereinafter sometimes referred to as "FGF-C1") in which a partial sequence at positions 41-83 in the amino acid sequence of the FGF1 protein was substituted with a partial sequence in the corresponding region in the amino acid sequence of the FGF2 protein, and another chimeric protein (which is referred to as "FGF-C(1(1/2)11) in Non-Patent Document 1 and hereinafter sometimes referred to as "FGF-C2") in which a partial sequence at positions 62-83 in the amino acid sequence of the FGF1 protein was substituted with a partial sequence derived from the FGF2 protein turned to be non-heparin-independent, although these chimeric proteins had a proliferation activity similar to that of FGF1. The present inventors then considered that the region at positions 62-83 in the amino acid sequence of FGF1 would be a region that determined the heparin dependence of FGF1. It is to be noted that the terms "position 62," "position 83," etc. as used in the present invention mean those positions on the amino acid sequence of FGF1 which are counted based on the definition that the N-terminus of an amino acid sequence corresponding to the full length cDNA of FGF1 represents an amino acid at position 1.

Thereafter, the present inventors confirmed that the chimeric protein FGF-C1 (FGF-C(1211)) had as strong a liver cell proliferation promoting activity and a neurite elongation promoting activity as FGF1 in the absence of heparin. The inventors then reported a medicinal composition for proliferating liver cells and a medicinal composition for proliferating nerve cells, and at the same time, they filed a patent application on then (Non-Patent Document 2 and Patent Document 1).

However, with regard to both chimeric proteins FGF-C1 and FGF-C2, two-thirds (2/3) or more of their overall about length is constituted with the amino acid sequence derived from FGF1. Accordingly, what was noted about the approach in the development of their use as a medicinal composition was that their heparin dependence was improved by substituting the heparin-dependence determining region of FGF1 with an FGF2-derived region and the purpose was no more than providing a medicinal composition as a substitute for FGF1. The possibility of the FGF2 substitute-containing medicinal composition has never been analyzed.

This time, the present inventors conducted intensive studies directed towards achieving the aforementioned object, namely, solving the problems of the FGF2 medicinal composition. During such studies, the inventors precisely determined the amino acid sequences of the previously produced large number of chimeric proteins, and they systematically measured the receptor binding specificity of each chimeric protein. As a result, the inventors have found that an FGF1/FGF2 chimeric protein (hereinafter sometimes simply referred to as a "chimeric protein") containing FGF-C(1211) and FGF-C(1(1/2)11) (hereinafter, the two FGFs are sometimes collectively referred to as "FGFC"), which are disclosed in the above-mentioned Non-Patent Document 1, is a protein retaining the same level of pharmacological activity as FGF2 and also having excellent pharmacological activity. The inventors have produced a FGF2 substitute-containing medicinal composition comprising the above-mentioned chimeric protein as an active ingredient. Specifically, they have produced a medicinal composition effective for wound healing and the prevention and treatment of radiation-induced damage, as well as an *in vitro* method in which such a medicinal composition is used for promoting the proliferation of stem cells; as a result they have completed the present invention.

The chimeric protein used as an active ingredient of the FGF2 substitute-containing medicinal composition described herein has an amino acid sequence comprising the amino acid sequence of the FGF1 protein in which a sequence of positions 41-83 or a partial sequence thereof including a sequence of at least positions 62-78 is substituted with a partial sequence at the corresponding positions in the amino acid sequence of the FGF2 protein; and the remaining region is formed of the amino acid sequence of FGF1.

The present invention relates to a medicinal composition which utilizes the following characteristics of the chimeric protein.
(1) The chimeric protein stimulates all subtypes of FGF receptor (as FGF1 does). As a result, the chimeric protein can also stimulate epithelial cells, which FGF2 cannot stimulate.
(2) The chimeric protein exhibits high activity without depending on heparin (similar to FGF2).
(3) The chimeric protein has a low adsorptive property onto the wall of a vessel (FGF1 and FGF2 have a high adsorptive property).
(4) The activity of the chimeric protein is highly stable from room temperature to body temperature (approximately 20°C to 40°C) (FGF1 and FGF2 have low stability).
(5) The chimeric protein has high resistance to trypsin decomposition (FGF1 and FGF2 are easily degradable).

The chimeric protein FGFC to be contained as an active ingredient in the medicinal composition described herein is a chimeric protein with FGF2, a two-thirds portion or more of which is constituted of an FGF1-derived protein. Despite such structure, it has been revealed that, as with FGF2, the chimeric protein FGFC is able to exhibit cell proliferating activity for ordinary cells without the addition of heparin. It has also been revealed that, as with FGF1, the chimeric protein is able to activate all subtypes of FGF receptors including FGFR2b, which FGF2 cannot stimulate. Thus, the present medicinal composition comprising the above-mentioned chimeric protein could be effectively used in application that require proliferation of epidermal keratinocytes and small intestine epithelial cells that require FGFR2b stimulation but the cell proliferation of which has so far been impossible to promote directly and effectively by FGF2. Moreover, since the present medicinal composition is able to exhibit its full activity without the addition of heparin, it could be effectively used in applications where heparin administration might cause adverse effects and in which the application of FGF1 has so far been discouraged. It can be said that this property is optimal for application to, for example, bleeding sites, such as wounded cutaneous keratinocytes and small intestine epithelial mucosal cells that are eroded due to radiation-induced damage.

Moreover, in comparison with FGF1 and FGF2, the chimeric protein FGFC has higher resistance to protease. Thus, when compared with FGF1, which is rapidly decomposed and inactivated in living bodies, the effective concentration of the chimeric protein FGFC just after administration is maintained for a long period of time. Hence, even if the chimeric protein FGFC is administered in a lower concentration, it can be anticipated to exhibit a comparable level of activity.

This chimeric protein is effective for the treatment of various types of diseases that involves or is expected to cause the proliferation of epithelial cells, as exemplified by the promotion of the regeneration of a wounded skin or subcutaneous tissues, and the improvement of wound healing when the natural healing ability of the skin is decreased. Also, this chimeric protein exhibits the action to promote the survival and proliferation of intestinal canal epithelial cells or bone marrow cells when such intestinal cells or bone marrow cells are damaged by exposure to radiation rays. Thus, it is useful for the treatment of various types of diseases that involves or is expected to cause the survival and proliferation of the aforementioned cells, as exemplified by the prevention and treatment of intestinal inflammation generated as side effects of radiotherapy for cancer and serious disorder of the intestinal canal or bone marrow in victims of nuclear accident. Furthermore, in other fields in which the effectiveness of the FGF2 medicinal composition has been confirmed or expected, a part or all of FGF2 may be substituted as a medicinal composition having a pharmacological effect equivalent to or higher than the FGF2. Examples include a medicinal composition for the promotion of the proliferation of stem cells, the treatment of bone fracture, application to regenerative medicine, the treatment of periodontal disease, the treatment of knee joint pain in chronic rheumatoid arthritis, the treatment of intractable skin ulcer, the treatment of coronary disease such as myocardial infarction or peripheral, circulatory failure such as intermittent claudication by means of utilizing strong vascularization action, and the recovery of lost functions (regenerative medicine), such as bone regeneration and nerve regeneration.

In the aforementioned applications, since FGFC is highly stable under temperature conditions from room temperature to body temperature (approximately 20°C to 40°C) and is also highly resistant to protease at 37°C, it can exhibit high activity. That is to say, the use of FGFC can minimize the influence of inactivation caused by protease contained in effusion generated due to various failures in living bodies.

Furthermore, FGFC has an excellent property as a medicinal preparation in that its concentration in a solution will not decrease rapidly when during storage in a vessel. Thus, a stable highly-active medicinal composition can be provided.

Specifically, the present invention includes the following features.
[1] An FGF2 substitute-containing medicinal composition, which comprises, as an active ingredient, an FGF1/FGF2 chimeric protein, wherein the amino acid sequence constituting the chimeric protein is shown in any one of SEQ ID NOS: 5 to 8, for use in the promotion of wound healing accompanied by the promotion of the epithelial cell proliferation.
[2] An FGF2 substitute-containing medicinal composition, which comprises, as an active ingredient, an FGF1/FGF2 chimeric protein, wherein the amino acid sequence constituting the chimeric protein is shown in any one of SEQ ID NOS: 5 to 8 for use in preventing and/or treating radiation-induced damage to the intestinal canal.
[3] The FGF2 substitute-containing medicinal composition for use according to [2] above, which is for use in the prevention or treatment of:
   (a) intestinal inflammation generated as side effect of radiotherapy for cancer; or
   (b) serious disorder of the intestinal canal in victims of nuclear accident;
   wherein the canal epithelial cells have been damaged by exposure to radiation rays.
[4] An FGF2 substitute-containing medicinal composition which comprises, as an active ingredient, an FGF1/FGF2 chimeric protein, wherein the amino acid sequence constituting the chimeric protein is shown in any one of SEQ ID NOS: 5 to 8 for use in preventing and/or treating radiation-induced damage to the bone marrow.
[5] An *in vitro* method of promoting the proliferation of stem cells, said method comprising contacting said stem cells with an FGF2 substitute-containing medicinal composition which comprises, as an active ingredient, an FGF1/FGF2 chimeric protein, wherein the amino acid sequence constituting the chimeric protein is shown in any one of SEQ ID NOS: 5 to 8.
[6] The FGF2 substitute-containing medicinal composition for use according to any of [1] to [4] above, wherein
   the chimeric protein is an active form of a chimeric protein obtained by culturing *Escherichia coli* transformed with DNA encoding the amino acid sequence shown in any one of SEQ ID NOS: 5 to 8, disrupting the cultured cells, and directly performing isolation and purification on a soluble fraction of the disrupted culture product.
[7] The FGF2 substitute-containing medicinal composition for use according to any one of [1] to [4] or [6] above, which is a medicinal composition exhibiting a higher pharmacological action than a medicinal composition comprising the FGF2 protein as an active ingredient.
[8] The *in vitro* method according to [5] above wherein the chimeric protein is an active form of a chimeric protein obtained by culturing *Escherichia coli* transformed with DNA encoding the amino acid sequence shown in any one of SEQ ID NOS: 5 to 8, disrupting the cultured cells, and directly performing isolation and purification on a soluble fraction of the disrupted culture product.
[9] The *in vitro* method according to [5] or [8] above wherein said medicinal composition exhibits a higher pharmacological action than a medicinal composition comprising the FGF2 protein as an active ingredient.

### Advantages of the Invention

A medicinal composition comprising the chimeric protein disclosed herein as an active ingredient is used as a substitute for a medicinal composition comprising FGF2 as an active ingredient; this provides an FGF2 substitute-containing medicinal composition **characterized in that** it requires no combined use of heparin to exhibit an activity of promoting the proliferation of fibroblasts and stem cells for which FGF2 exhibits effectiveness and that it also has an activity of promoting the proliferation of epithelial cells. For example, there can be provided an agent for promoting wound healing, a medicinal composition for preventing and treating radiation-induced damage to the intestinal epithelia and the bone marrow. Also provided is an *in vitro* method in which such a medicinal composition is used for promoting the proliferation of stem cells.

Moreover, the present FGF2 substitute-containing medicinal composition has excellent properties in that it has higher stability to temperature and protease than the conventional FGF2 medicinal composition and that its concentration in a solution will not decrease rapidly during storage in a vessel, which makes it easy to formulate.

### Brief Description of the Drawings

Figure 1 shows the receptor specificity of a chimeric protein that can be measured based on cell proliferation promoting activity (in the presence of heparin);
Figure 2 shows cell proliferation promoting activity on BaF3 cells in which FGFR1c was forcibly expressed (in the presence of heparin);
Figure 3 shows cell proliferation promoting activity on BaF3 cells in which FGFR2b was forcibly expressed (in the presence of heparin);
Figure 4 shows the receptor specificity of a chimeric protein that can be measured based on cell proliferation promoting activity (in the absence of heparin);
Figure 5 shows the resistance of a chimeric protein to decomposition by trypsin (in comparison with FGF1);
Figure 6 shows the resistance of a chimeric protein to decomposition by trypsin (in comparison with FGF2);
Figure 7 shows the resistance of a chimeric protein to decomposition by trypsin (in comparison with FGF1 and FGF2);
Figure 8 shows the resistance of a chimeric protein to decomposition by trypsin (time dependence);
Figure 9 shows the stability of the activity of a chimeric protein during storage at 37°C (body temperature) (in comparison with FGF1);
Figure 10 shows the stability of the activity of a chimeric protein during storage at 37°C (body temperature) (in comparison with FGF2);
Figure 11 shows the stability of the concentration of a chimeric protein in solution during storage in a vessel;
Figure 12 shows the structural stability of a chimeric protein in solution (at 25°C);
Figure 13 shows the structural stability of a chimeric protein in solution (temperature dependence);
Figure 14 shows the activity (1) of a chimeric protein to promote the proliferation of epidermal cells which is an important step in wound healing;
Figure 15 shows the activity (2) of a chimeric protein to promote the proliferation of epidermal cells which is an important step in wound healing;
Figure 16 shows the activity of a chimeric protein to promote the proliferation of fibroblasts;
Figure 17 shows the activity of a chimeric protein to promote the prevention and treatment of radiation-induced damage to living bodies;
Figure 18 shows an efficient mass production of a soluble chimeric protein using an *Escherichia coli* expression system; and
Figure 19 shows the activity of a chimeric protein to promote wound healing.

### Best Mode for Carrying Out the Invention

The amino acid sequence of a chimeric protein contained as an active ingredient in the medicinal composition described herein comprises the amino acid sequence of the FGF1 protein in which a partial sequence including a sequence of at least positions 62-78 within a sequence of positions 41-83 is substituted with a partial sequence at the corresponding positions in the amino acid sequence of the FGF2 protein; and the remaining region is formed of the amino acid sequence of FGF1. That is to say, cDNAs encoding FGF1 and FGF2 are prepared using a cassette format, and a chimeric body is then prepared with regard to cDNA. Thereafter, the chimeric body is expressed using an expression system such as *Escherichia coli,* yeast or animal cells so as to obtain the aforementioned chimeric protein.

In addition, the chimeric protein described herein is typically processed into a medicinal preparation by purifying an expression product from the aforementioned host cells. After the production of chimeric cDNA, it can be applied to gene therapy, using an expression vector that can be administered to humans or animals to be treated.

As an FGF1 protein and an FGF2 protein used as bases of the aforementioned chimeric protein, FGF from all types of mammals such as a human, a mouse, a rat, a bovine, or a horse may be used. In order to avoid undesirable reactions caused by an immune system, a mammal of the same origin as the animal to be treated is preferable.

The amino acid sequence constituting the chimeric protein described herein is basically composed of the amino acid sequence of the FGF1 protein, and the amino acid sequence portion thereof associated with heparin dependence is substituted with the corresponding sequence portion of the FGF2 protein. Specifically, a partial sequence containing a sequence of at least positions 62-78 in a partial sequence of positions 41-83 in the amino acid sequence of the FGF1 protein may be substituted with a partial sequence at the corresponding positions in the amino acid sequence of the FGF2 protein. The chimeric protein described herein preferably has the amino acid sequence of the FGF1 protein wherein the partial sequence at positions 41-78 in the amino acid sequence has been entirely substituted with the corresponding amino acid sequence of the FGF2 protein (which corresponds to the amino acids at positions 44-81). Moreover, the aforementioned chimeric protein may comprise an addition, deletion, substitution, or modification in a portion of its amino acid sequence as long as it is able to exhibit its functions.

Typical examples of the chimeric protein FGFC described herein include the chimeric proteins FGF-C(1211) and FGF-C(1(1/2)11) described in Non-Patent Document 1. Now, the types of amino acid sequences of the known chimeric compounds, namely, the chimeric proteins FGF-C(1211) and FGF-C(1(1/2)11) described in Non-Patent Document 1, will be analyzed.

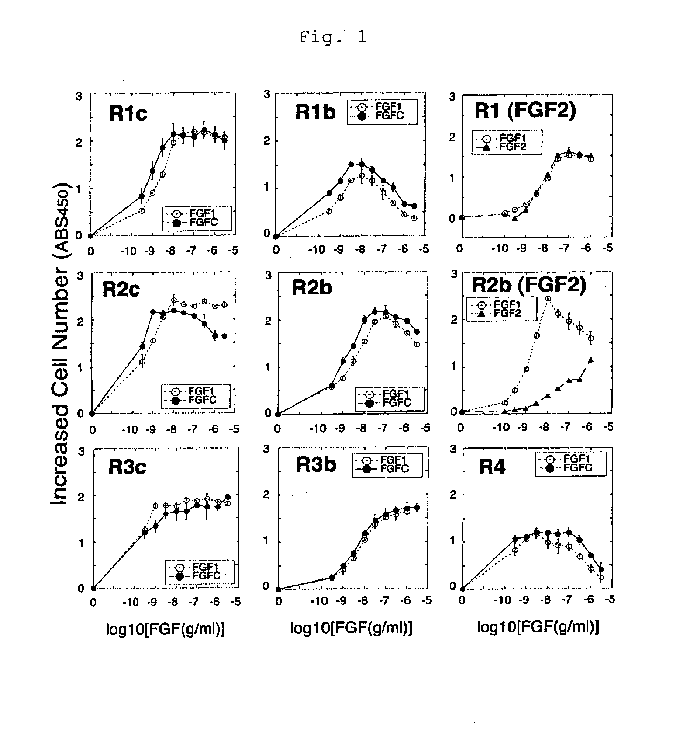

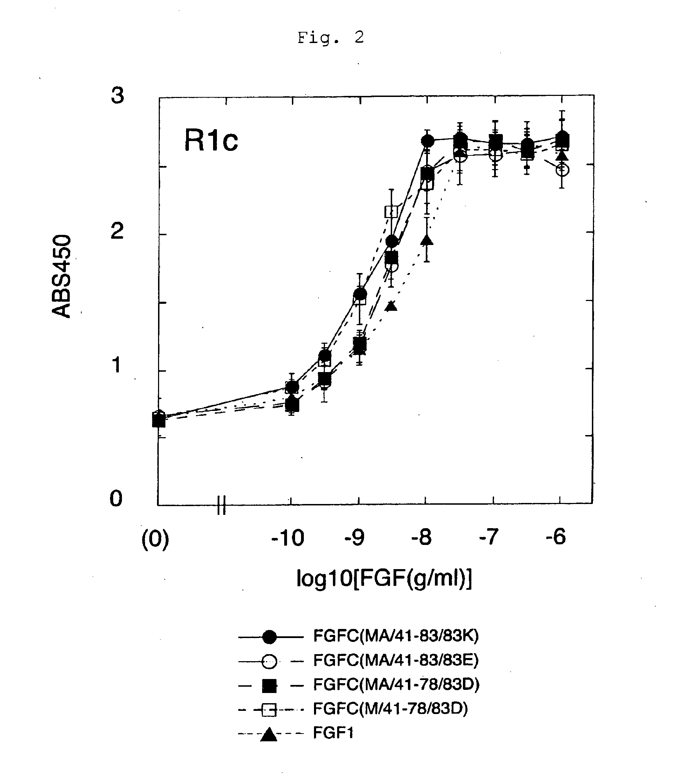

In Non-Patent Document 1, a synthesis method using a cassette system described in Non-Patent Document 10 was adopted. As shown in Figure 1A, the position 83 of each of the aforementioned FGF-C1 and FGF-C2 is substituted with the amino acid sequence of FGF2. Thus, in the original design, Lys(K) at the corresponding position of FGF2 is adopted. However, according to the cassette-system design, the connected portion of the second cassette with the third cassette which corresponds to the position 83 is a restriction enzyme Nco1 site, and thus a mutation of Glu(E) is designed to be introduced. Furthermore, the same publication (page 126, left column, line 13 to right column, line 7) describes an oligonucleotide sequence set constituting "Cassette B (pTI2X, Figure 2)" for producing the FGF-C(1211) chimeric protein shown in Figure 1B. Cassette B is produced using such oligonucleotide sequence, and this Cassette is then exchanged with the homologous Cassette portion of an FGF1 gene by the method described in Non-Patent Document 10, so as to obtain a chimeric protein as a gene expression product. In the case of this chimeric protein, the amino acid at position 83 can be Asp(D), which is identical to the amino acid at position 83 of FGF1. Similarly, in the case of the FGF-C(1(1/2)11) chimeric protein as well, the use of an Munl-Ncol cassette has been proposed. Thus, as in the case of producing the FGF-C(1211) chimeric protein using the "Cassette B," chimeric proteins comprising 3 types of amino acids, Lys(K), Glu(E) and Asp(D), as the amino acid at position 83, are also included.

In other words, Non-Patent Document 1 substantially discloses three types of chimeric proteins as FGF-C(1211) and FGF-C(1(1/2)11) wherein the amino acid at position 83 is Lys(K), Glu(E) or Asp(D).

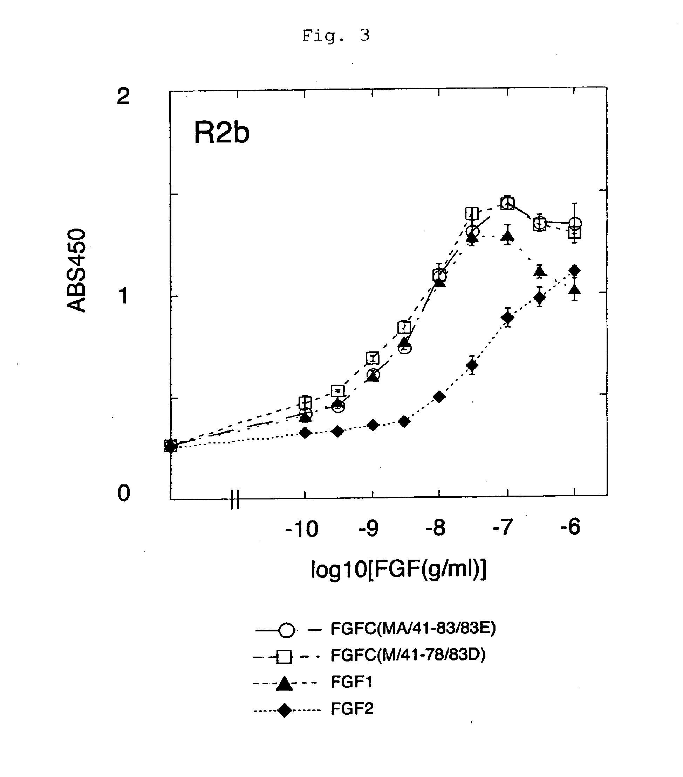

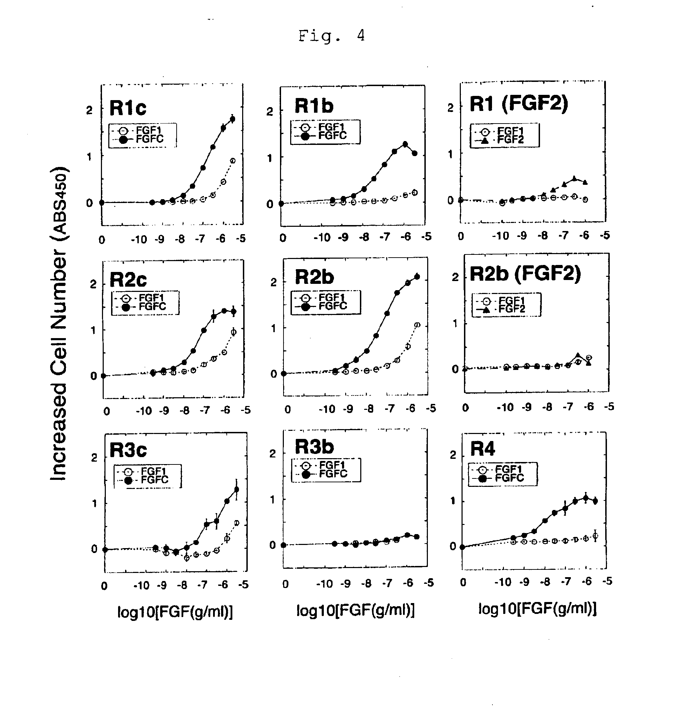

However, in this Non-Patent Document 1, the amino acid at position 83 that is Lys(K) or Glu(E) has merely been proposed. What was actually produced was a chimeric protein as an expression product using the aforementioned oligonucleotide sequence. Thus, the amino acid at position 83 of the produced chimeric protein was Asp(D) derived from FGF1. In Figure 3 and Figure 4 of the same publication, it was also Asp(D) that was measured in terms of heparin-dependent DNA synthesis promoting activity and heparin binding strength. When this amino acid at position 83 is Asp(D) derived from FGF1, the amino acid sequence at positions 79-82 upstream of the position 83 is shared by FGF1 and FGF2 (see Figure 1A of the same publication). Accordingly, the chimeric protein of interest can also be described as a chimeric protein based on FGF1 wherein the amino acid sequence at positions 41-78 has been substituted with the amino acid sequence derived from FGF2.

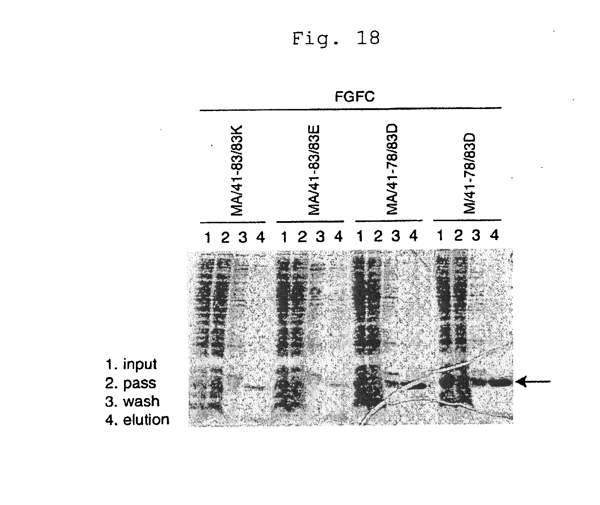

Further, when such chimeric proteins are produced, the amino acids ranging from the N-terminus to position 21 of the full-length translation product of FGF1 cDNA are such that the deletion of 21 amino acids at the N-terminus leads to a high expression level and provides greater ease in handling, as in the case of an abbreviated isoform obtained during the extraction of the FGF1 protein from animal tissues. Modification, such as the addition of MetAla for translation and the posttranslational cleavage of methionine during the production of the N-terminal side in *Escherichia coli,* is also a common practice. It has already been known that the activity of FGF1 is not affected by such difference in the N-terminus. Accordingly, the term "FGF-C(1211)," "FGF-C(1(1/2)11)," or simply, "FGFC" is used herein to include a full-length protein comprising the 21 amino acids at the N-terminus, an abbreviated isoform with a deletion of the 21 amino acids, and a truncated form comprising an addition of MetAla(MA) to the N-terminus of the abbreviated form. However, abundant expression in an *Escherichia coli* host is intended, an abbreviated form in which the N-terminus has been deleted or a truncated form in which MetAla(MA) has been added to the N-terminus is preferable because of high expression level and high solubility. In particular, a chimeric protein having Asp(D) at position 83 is most preferable because it can be easily isolated and purified as an active form (that is not an inclusion body but is precisely folded) from a soluble fraction of the mass of cells disrupted after culturing the transformed *Escherichia coli* (see Example 11 and Figure 18).

The FGFC chimeric protein used in Examples 1-12 is FGFC(MA/41-78/83D) having the amino acid sequence shown in SEQ ID NO: 5 wherein the amino acid at position 83 is Asp(D) and which was actually produced in Non-Patent Document 1, too. In Example 13 and subsequent Example, other typical FGFC chimeric proteins wherein the amino acid at position 83 was Lys(K) or Glu(E) were also synthesized separately and a comparison was made among the 3 types of chimeric proteins in terms of FGF receptor-stimulating activity and other properties. All of these chimeric proteins had higher activities to stimulate various FGF receptors than FGF1 and FGF2 in the presence of heparin. In addition, the excellent properties of these chimeric proteins such as stability of concentration in solution during storage in a vessel, temperature stability, and resistance to trypsin decomposition, were observed. Among others, a chimeric protein wherein the amino acid at position 83 was Asp(D) (SEQ ID NO: 7: FGFC(MA/41-78/83D) was particularly excellent in terms of all of the above-mentioned aspects. In addition, Lys(K) at position 83 is an amino acid derived from FGF2, and Asp(D) is an amino acid derived from FGF1. However, Glu(E) at position 83 is an exogenous amino acid. Thus, when it is used in a medicinal composition, it may be recognized as a foreign substance, and so, it is inappropriate.

As described above, the chimeric proteins (SEQ ID NOS: 5 and 7) wherein the amino acid at position 83 is Asp(D) are also referred to as chimeric proteins wherein the amino acid sequence at positions 41-78 of FGF1 has been substituted with an amino acid sequence derived from FGF2.

Hereinafter, a method for preparing the chimeric protein disclosed herein will be specifically described.

As stated above, the chimeric protein disclosed herein can be prepared by the method described in Non-Patent Document 10, using the oligonucleotide for the production of FGF-C(1211) disclosed in Non-Patent Document 1. If desired, the following methods can also be applied:
(a) With regard to FGF1 and FGF2 cDNAs or artificially modified products thereof, a DNA fragment may be excised from either one of the FGF cDNAs using a suitable restriction enzyme. Alternatively, a new DNA fragment may be produced by a method such as PCR, and a restriction enzyme terminus may be excised. Thereafter, the DNA fragment may be ligated to a suitable site of the other FGF cDNA using DNA ligase. In this case, an oligonucleotide may be inserted for matching a reading frame, or a part of nucleotide sequence may be modified for creating the same restriction enzyme site. For example, (b) a DNA fragment may be excised from one FGF cDNA with a suitable restriction enzyme, and it may be then bound using DNA ligase to a site obtained by cleaving the homologous site of the other cDNA with the same restriction enzyme. That is, DNA fragments encoding the corresponding regions in a case in which the sequences of FGF1 and FGF2 are aligned based on amino acid homology are replaced by each other. One or two or more types of restriction enzymes are used herein as restriction enzymes. Basic operations of PCR for site-directed mutagenesis are carried out in accordance with a method previously published by the present inventors [Imamura, T. et al., Science 249, 1567-1570 (1990)].

The type of a plasmid into which DNA is to be incorporated is not particularly limited as long as it can be replicated and maintained in a host. Examples of such plasmid include *Escherichia coli*-derived pBR322 and pUC18, and pET-3c constructed based on the aforementioned plasmids.

An example of a method for incorporating DNA into a plasmid is a method described in T. Maniatis et al., Molecular Cloning, Cold Spring Harbor Laboratory, p.239 (1982).

The cloned gene is ligated downstream of a promoter in a vector suitable for expression, so as to obtain an expression vector. Examples of such vector include: the aforementioned *Escherichia coli*-derived plasmids (pBR322, pBR325, pUC12, pUC13, and pET-3); *Bacillus subtilis-*derived plasmids (pUB110, pTP5, and pC194), yeast-derived plasmids (pSH19 and pSH15); bacteriophages such as λ phage and derivatives thereof; animal viruses such as retrovirus and vaccinia virus; and insect viruses.

The FGFC gene of the present invention has a translation start codon ATG at the 5'-terminus, and also has a translation stop codon, TAA, TGA or TAG at the 3'-terminus thereof. Further, in order to express the gene, a promoter is connected with a site upstream thereof. The type of a promoter used in the present invention is not particularly limited as long as it is a suitable promoter compatible with a host used in the expression of the gene.

When a host to be transformed is *Escherichia coli,* a trp promoter, a lac promoter, a recA promoter, a λPL promoter, an 1pp promoter or a T7 promoter is preferable. When the host is *Bacillus subtilis,* an SP01 promoter, an SP02 promoter or a penP promoter is preferable. When the host is yeast, a PHO5 promoter, a PGK promoter, a GAP promoter or an ADH promoter is preferable. When the host is an animal cell, an SV40-derived promoter or a retrovirus promoter is preferable.

Using the thus constructed vector comprising recombinant DNA having a nucleotide sequence encoding a chimeric protein, a transformant containing the vector is produced.

Examples of a host that can be used herein include *Escherichia coli* [e.g. BL21, BL21(DE3), BL21(DE3)pLysS, and BL21(DE3)pLysE], *Bacillus subtilis* (e.g. *Bacillus subtilis* DB105), yeast (e.g. *Pichia pastoris* and *Saccharomyces cerevisiae*)*,* animal cells (e.g. COS cells, CHO cells, BHK cells, NIH3T3 cells, BALB/c3T3 cells, HUVE cells, and LEII cells), and insect cells.

The aforementioned transformation is carried out by a method commonly used with respect to each host. Even if it is not a common method, it is adequate if it is applicable. For example, if the host is *Escherichia coli,* a vector comprising recombinant DNA is introduced by a temperature shock method or an electroporation method into competent cells that have been produced by a calcium method or other methods. If the host is yeast, a vector comprising recombinant DNA is introduced by a temperature shock method or an electroporation method into competent cells that have been produced by a lithium method or other methods. If the host is an animal cell, a vector comprising recombinant DNA is incorporated into cells at a growth phase by a calcium phosphate method, a lipofection method, or an electroporation method.

Thus, a transformant having a vector comprising recombinant DNA having a nucleotide sequence encoding a chimeric protein is obtained. The transformant is cultured in a medium to generate a chimeric protein.

When the transformant is cultured, a medium commonly used with respect to each host is used in the culture. Even if the medium is not a common one, it is adequate if it is an applicable medium. For example, if the host is *Escherichia coli,* an LB medium may be used. If the host is yeast, a YPD medium may be used. If the host is an animal cell, a medium formed by adding animal serum to Dulbecco's MEM, may be used. The culture is carried out under conditions commonly applied with respect to each host. Even if such conditions are not common ones, it is adequate if the conditions are applicable. For example, if the host is *Escherichia coli,* the culture may be carried out at approximately 30°C to 37°C for approximately 3 to 24 hours. If necessary, aeration or stirring may be carried out during the culture. If the host is yeast, the culture may be carried out at approximately 25°C to 37°C for approximately 12 hours to 2 weeks. If necessary, aeration or stirring may be carried out during the culture. If the host is an animal cell, the culture may be carried out at approximately 32°C to 37°C in 5% CO₂ at a humidity of 100% for approximately 24 hours to 2 weeks. If necessary, conditions for the gaseous phase may be altered, or stirring may be carried out during the culture.

To extract a chimeric protein from the aforementioned culture product such as a cultured cell mass or cultured cells, the cell mass or cells after completion of the culture are disrupted by a homogenizer, French press, sonication, lysozyme and/or freezing-and-thawing, so that a protein of interest is eluted out of the cell mass, and a chimeric protein is then obtained from a soluble fraction. When such chimeric protein of interest is contained in an insoluble fraction, the following method may also be applied. That is, the cell mass or cells are disrupted, and the insoluble fraction is then recovered by centrifugation. Thereafter, the insoluble fraction is solubilized using a buffer containing guanidine hydrochloride, and a chimeric protein is then recovered from a soluble fraction. In addition to these methods, there is also a method, which comprises directly disintegrating the cell mass or cells using a buffer containing a protein denaturant such as guanidine hydrochloride, and then eluting a chimeric protein of interest out of the cell mass.

The chimeric protein can be purified from the aforementioned supernatant by appropriately combining known separation and purification methods. Examples of such known separation and purification methods that can be used herein include salting-out, solvent precipitation, dialysis, ultrafiltration, gel filtration, SDS-polyacrylamide gel electrophoresis, ion-exchange chromatography, affinity chromatography, reverse phase high performance liquid chromatography, and isoelectric focusing. Moreover, an affinity chromatography method using heparin sepharose as a carrier can be applied to many FGF chimeric proteins.

The thus obtained sample is refrigerated at 4°C or lower. It can also be frozen at -20°C or lower as long as the activity of the chimeric protein is not impaired. Further, the sample may be subjected to dialysis and freeze-drying, so that it may be processed into a dry powder.

An FGF2 substitute-containing medicinal composition which comprises the thus obtained chimeric protein as an active ingredient can be used as a medicinal composition having pharmacological effects equivalent to or greater than those of all medicinal compositions for which an FGF2 medicinal composition is conventionally used or is planned to be used. Examples of the aforementioned medicinal composition include: an agent for promoting wound healing; a medicinal composition for preventing or treating radiation-induced damage to various organs including the intestinal epithelia and the bone marrow; and a medicinal composition for promoting the proliferation of stem cells.

In particular, the present medicinal composition is useful for the treatment of various types of diseases that involves or is expected to cause the proliferation of epithelial cells, as exemplified by the promotion of the regeneration of a wounded skin or subcutaneous tissues, and the improvement of wound healing when the natural healing ability of the skin is decreased. The above-mentioned chimeric protein is also effective for the improvement of a disorder of the intestinal canal or bone marrow cells damaged by exposure to radiation rays. This chimeric protein exhibits the action to promote the survival and proliferation of intestinal canal epithelial cells or bone marrow cells. Thus, it is useful for the treatment of various types of diseases that involves or is expected to cause the survival and proliferation of the aforementioned cells, as exemplified by the prevention and treatment of intestinal inflammation generated as side effects of radiotherapy for cancer, serious disorders of the intestinal canal or bone marrow of victims of nuclear accident.

Furthermore, because of the characteristics of the chimeric protein of the present invention, the FGF2 substitute-containing medicinal composition which comprises the chimeric protein of the present invention as an active ingredient is highly stable under temperature conditions of room temperature (25°C) in comparison with FGF1. In addition, the present medicinal composition is highly resistant to protease under conditions of body temperature (37°C). Thus, the present medicinal composition can minimize the influence of inactivation caused by protease contained in effusion generated due to various failures in living bodies. Further, the present medicinal composition has an excellent property as a medicinal preparation in that its concentration in a solution will not decrease ragidly during storage in a vessel. Thus, a stable highly-active medicinal composition can be provided.

The FGF2 substitute-containing medicinal composition which comprises the chimeric protein of the present invention as an active ingredient is formulated into a medicinal composition such as a liquid an injection, powder, granules, a tablet, a suppository, an enteric coating agent or a capsule according to an ordinary method for producing preparations using a pharmaceutically acceptable solvent, excipient, carrier or adjuvant. The content of the chimeric protein as an active ingredient in the medicinal composition may be set at approximately 0.000001% to 1.0% by weight. The present medicinal composition can be safely administered as a liver cell proliferating agent or a nerve cell differentiation survival promoting agent to a mammal such as a human, a mouse, a rat, a rabbit, a dog or a cat via a parenteral or oral administration route. The dose of the present medicinal composition may be appropriately changed depending on the dosage form, the administration route and symptoms. In a case in which the medicinal composition is administered to a mammal such as a human, the chimeric protein may be administered at a dose of 0.01 to 10 mg/1 kg of body weight per day.

### Examples

Hereinafter, the present invention will be described in more detail by the following examples. It is to be noted that the term "FGFC" as simply used in the following examples and drawings to mean "FGFC (MA/41-78/83D)" which is a truncated form of a typical chimeric protein of the present invention wherein Asp(D) is at position 83.

### [Example 1]

### Receptor specificity of chimeric protein that can be measured based on cell proliferation promoting activity (in the presence of heparin)

(1-1) The receptor specificity of FGFC was compared with the receptor specificity of FGF1 and that of FGF2. A cell strain BaF3 which had neither endogenous FGF receptors nor endogenous heparan sulfate was used as a parent strain. The BaF3 cells were forced to express each of 7 representative subtypes of FGF receptor (FGFR1c, FGFR1b, FGFR2c, FGFR2b, FGFR3c, FGFR3b, and FGFR4) to produce respective cell strains. If the forcibly expressed FGF receptor is stimulated, the cells start to proliferate. Thus, receptor-stimulating activity can be measured by measuring the increased cell number. It is to be noted that such cell number was measured by colorimetrically assaying the activity of mitochondrial enzyme proportional to the cell number.

The BaF3 cells were suspended in an RPMI1640 medium containing 10% FBS, and the suspension was then dispersed on a 96-well microtiter plate (1 × 10⁴ cells/well). Thereafter, heparin was added to give a concentration of 5 µg/ml. FGF samples were added in various concentrations. After completion of the culture for 44 hours, a TetraColorOne reagent was added to the culture, and the obtained mixture was further cultured for 4 hours. Thereafter, the absorbance at 450 nm was measured. The results are shown in Figure 1. Each point indicates the mean +/- standard deviation (S.D.) of triplicate samples.

It was demonstrated that, as with FGF1, FGFC had an activity capable of stimulating all of the FGF receptor subtypes (FGFR1c, FGFR1b, FGFR2c, FGFR2b, FGFR3c, FGFR3b, and FGFR4) in the presence of heparin, and that the strength of the activity was equivalent to or slightly stronger than the activity of FGF1. On the other hand, it was demonstrated that the activity of FGF2 on the receptor FGFR2b which was particularly important for the promotion of the proliferation of epithelial cells was extremely weak, and that such activity was approximately one-thousands of FGF1 (Figure 1).

That is to say, as shown in Figure 1, FGFC is able to activate all of the FGF receptor subtypes including FGFR2b unstimulable by FGF2 at a level equivalent to or higher than FGF1. These results demonstrate that FGFC may enable the production of a medicinal composition that can be effectively used in applications that require the proliferation of epidermal keratinocytes, small intestine epithelial cells, etc. that require FGFR2b stimulation but the cell proliferation of which has so far been impossible to directly promote and effectively by FGF2.
(1-2) The cell proliferation promoting activity on BaF3 cells forced to express FGFR1c was examined for FGFC(MA/41-83/83K), FGFC(MA/41-83/83E), FGFC(MA/41-78/83D), FGFC(M/41-78/83D) and FGF1 in the presence of exogenous heparin under the same conditions as those described in (1-1) above (Figure 2). As shown in Figure 2, it was demonstrated that FGFC(MA/41-83/83K), FGFC(MA/41-83/83E), FGFC(MA/41-78/83D), and FGFC(M/41-78/83D) had the activity of stimulating the receptor FGFR1c at a level equivalent to or slightly stronger than FGF1 in the presence of exogenous heparin.
(1-3) The cell proliferation promoting activity on BaF3 cells forced to express the receptor FGFR2b important for the promotion of the proliferation of epithelial cells was examined for FGFC(MA/41-83/83E), FGFC(M/41-78/83D), FGF1 and FGF2 in the presence of exogenous heparin under the same conditions as those described in (1-1) above (Figure 3). As shown in Figure 3, it was demonstrated that FGFC(MA/41-83/83E) and FGFC(MA/41-78/83D) had the activity of stimulating the receptor FGFR2b at a level equivalent to or slightly stronger than FGF1 in the presence of exogenous heparin. On the other hand, it was demonstrated that FGF2 had an extremely weak activity on the receptor FGFR2b, approximately several tens of times less than that on FGF1.

### [Example 2]

### Receptor specificity of chimeric protein that can be measured based on cell proliferation promoting activity (in the absence of heparin)

FGFC, FGF1 and FGF2 were examined in terms of receptor specificity. The experiment was carried out under almost the same conditions as those in Example (1-1). In this experiment, however, heparin was not added. Each point indicates the mean +/- standard deviation (S.D.) of triplicate samples.

It was demonstrated that FGFC had the activity of stimulating almost all of the FGF receptor subtypes (FGFR1c, FGFR1b, FGFR2c, FGFR2b, FGFR3c, and FGFR4) even in the absence of heparin. In addition, FGF2 did not have such activity on any of the receptor subtypes examined (FGFR1c and FGFR2b) (Figure 4).

The data shown in Figure 4 was adjusted to have the same scale as that shown in Figure 1, and a comparison was made with respect to the receptors. As a result, FGF1 lost a majority of its receptor stimulating activity unless heparin was added. In contrast, FGFC retained high activity on the receptors other than R3b. In particular, it is worthy of attention that FGFC had extremely high R2b receptor stimulating activity even in the absence of heparin, whereas FGF2 did not have such activity whether or not heparin is present. Since the R2b receptor exists in a large amount in epithelial cells, this fact demonstrates that FGFC has an epithelial cell proliferating activity although FGF2 does not have an epithelial cell proliferating activity.

There may be a case in which FGF1 has the receptor stimulating activity even in the absence of heparin, if it is administered in a high concentration such as 1 mg/kg or more. However, the administration of such high concentration of FGF1 into living bodies is not realistic because it causes problems regarding immune system stimulation that result from the administration of high concentrations of polypeptide preparations as well as adverse effects on kidney function.

### [Example 3]

### Resistance of chimeric protein to trypsin decomposition (concentration dependence)

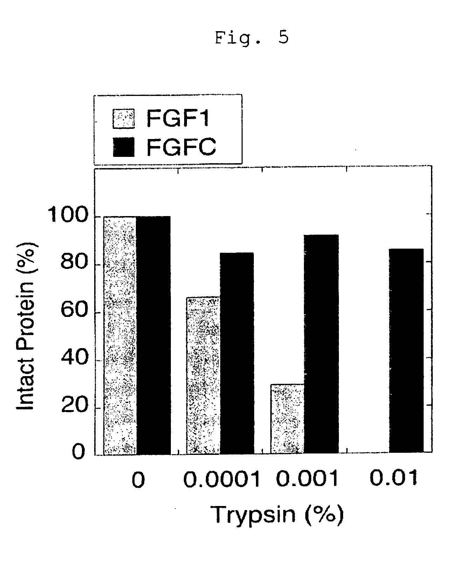

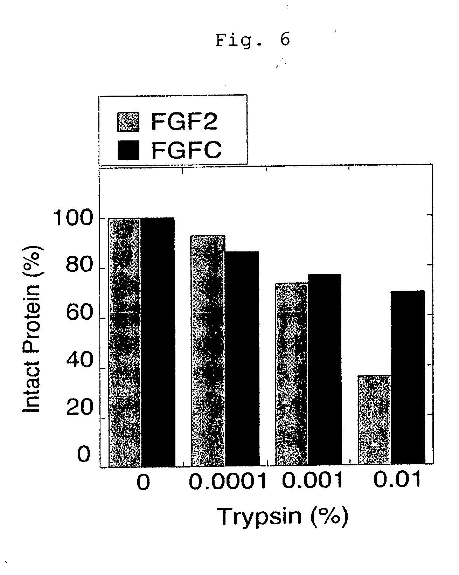

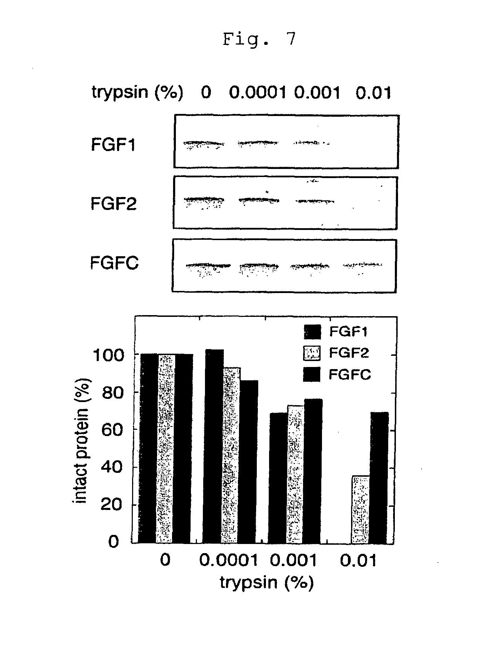

(3-1) The resistance of FGFC and FGF1 to decomposition by trypsin was examined. Trypsin was added to 30µl of PBS solution containing 500 ng of each FGF, resulting in various final concentrations. The obtained mixture was incubated at 37°C for 1 hour, so as to allow protein decomposition. Thereafter, the sample was separated by SDS-polyacrylamide electrophoresis. After completion of the electrophoresis, the gel was treated with a CBB staining solution that would stain protein in proportion to its amount. Thereafter, optical scanning was carried out to quantify the amount of the remaining FGF protein that had not been decomposed (Figure 5).
   As shown in Figure 5, approximately 80% of FGFC remained after the treatment with 0.01% trypsin. In contrast, FGF1 was completely decomposed. In addition, approximately 90% of FGFC remained after treatment with 0.001% trypsin. In contrast, only approximately 30% of FGF1 remained after the same treatment. These results demonstrate that FGFC can minimize the influence of inactivation caused by proteases which are contained in effusion generated due to various failures in living bodies.
(3-2) The resistance of FGFC and FGF2 to decomposition by trypsin was examined under the same conditions as those described in (3-1) above. Trypsin was added to 30µl of PBS solution containing 500 ng of each FGF, resulting in various final concentrations. The obtained mixture was incubated at 37°C for 1 hour, so as to allow protein decomposition. Thereafter, the sample was separated by SDS-polyacrylamide electrophoresis. After completion of the electrophoresis, the gel was treated with a CBB staining solution that would stain protein in proportion to its amount. Thereafter, optical scanning was carried out to quantify the amount of the remaining FGF protein that had not been decomposed (Figure 6).
   As shown in Figure 6, approximately 70% of FGFC remained after the treatment with 0.01% trypsin. In contrast, only approximately 35% of FGF2 remained. These results demonstrate that FGFC can minimize the influence of inactivation caused by protease contained in effusion generated due to various failures in living bodies.
(3-3) FGFC, FGF1, and FGF2 were subjected to an experiment under the same conditions as those described in (3-1) above and compared to each other for resistance to decomposition by trypsin. Trypsin was added to 30µl of PBS solution containing 500 ng of each FGF, resulting in various final concentrations. The obtained mixture was incubated at 37°C for 1 hour, so as to allow protein decomposition. Thereafter, the sample was separated by SDS-polyacrylamide electrophoresis. After completion of the electrophoresis, the gel was treated with a CBB staining solution that would stain protein in proportion to its amount. Thereafter, optical scanning was carried out to quantify the amount of the remaining FGF protein that had not been decomposed (Figure 7).

As shown in Figure 7, approximately 30% of FGFC remained after the treatment with 0.01% trypsin. In contrast, FGF1 was completely decomposed and 60% or more of FGF2 was decomposed. These results demonstrate that FGFC can minimize the influence of inactivation caused by protease contained in body fluid or the like. These results suggest that FGFC has a longer *in vivo* half-life than FGF1 and FGF2.

### [Example 4]

### Resistance of chimeric protein to decomposition by trypsin (time dependence)

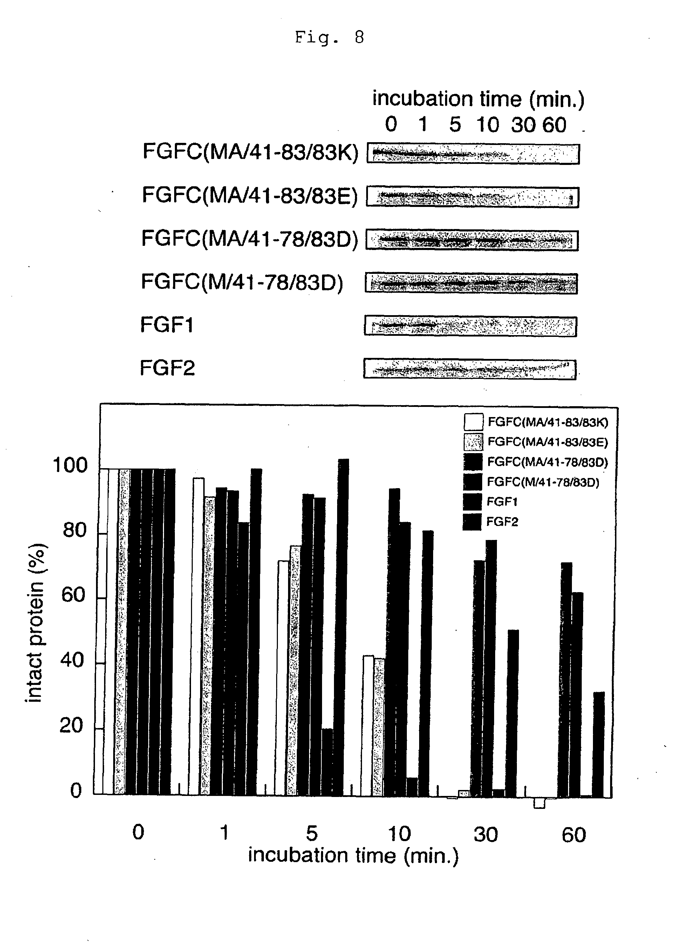

The resistance of FGFC(MA/41-83/83K), FGFC(MA/41-83/83E), FGFC(MA/41-78/83D), FGFC(M/41-78/83D), FGF1 and FGF2 to decomposition by trypsin were examined from the viewpoint of time dependence under the same conditions as those described in Example 3. Trypsin was added to 30µl of PBS solution containing 500 ng of each FGF, resulting in a final concentration of 0.015%. The obtained mixture was incubated at 37°C for various periods of time, so as to allow protein decomposition. Thereafter, the sample was separated by SDS-polyacrylamide electrophoresis. After completion of the electrophoresis, the gel was treated with a CBB staining solution that would stain protein in proportion to its amount. Thereafter, optical scanning was carried out to quantify the amount of the remaining FGF protein that had not been decomposed (Figure 8).

As shown in Figure 8, in the case of FGFC(MA/41-78/83D) and FGFC(M/41-78/83D), approximately 60%-70% of FGFC remained after the treatment with 0.015% trypsin for 60 minutes. In contrast, in the case of FGFC(MA/41-83/83K), FGFC(MA/41-83/83E) and FGF1, they were completely decomposed, and in the case of FGF2, only approximately 30% remained. By the treatment with 0.015% trypsin for 5 minutes, only approximately 20% of FGF1 remained, whereas in the case of FGFC(MA/41-78/83D) and FGFC(M/41-78/83D), 90% or more FGFC remained. These results demonstrate that FGFC(MA/41-78/83D) and FGFC(M/41-78/83D) can minimize the influence of inactivation caused by protease contained in effusion generated due to various failures in living bodies. These results suggest that FGFC has a longer *in vivo* half-life than FGF1 and FGF2.

### [Example 5]

### Stability of activity of chimeric protein during storage under body temperature conditions (37°C)

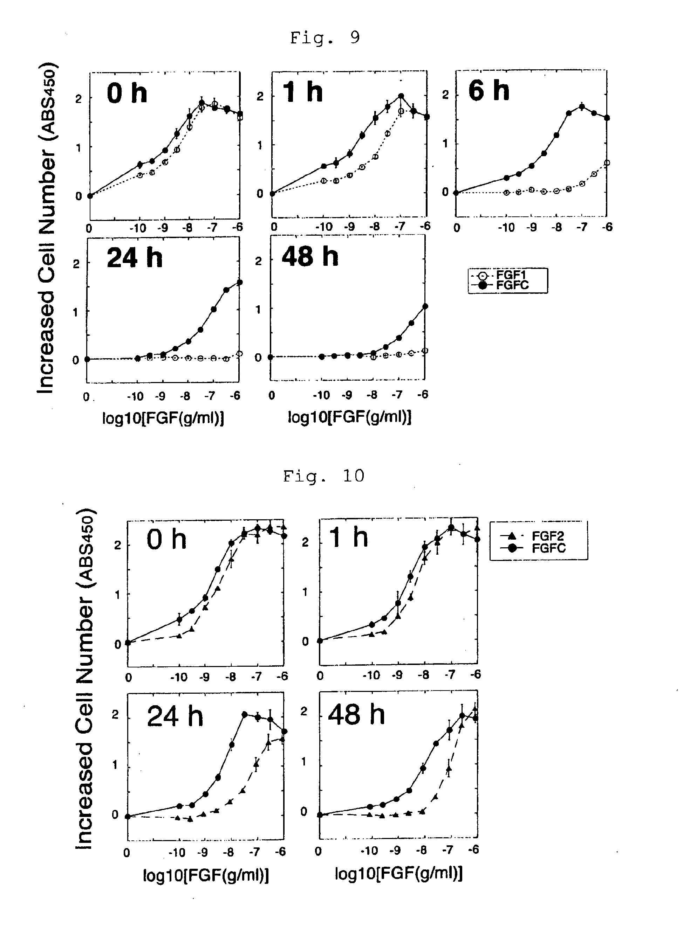

(5-1) The stability of FGFC and FGF1 during storage at 37°C was examined. In order to reduce the loss caused by protein adsorption, 5 µg of FGF was dissolved in 50 µl of RPMI1640 medium containing 10% BSA, and it was then stored in an Eppendorf tube at 37°C for various periods of time. Thereafter, using the thus stored solution as a stock solution, various types of diluted solutions were prepared. The biological activity of such diluted solution on FGFR1c/BaF3 cells was measured. The measurement method was the same as that described in Example 1(Figure 9).
   The concentration plotted on the horizontal axis in Figure 9 represents the initial concentration. The results demonstrated the following. FGFC and FGF1 initially exhibited almost the same level of activity. However, under temperature conditions of approximately body temperature (37°C), FGF1 started to lose its activity in only about one hour after the initiation of the experiment, and almost no activity remained after 6 hours. In contrast, in the case of FGFC, the activity was little influenced by the 1-6 hour storage.
(5-2) The stability of the activity of FGFC and FGF2 during storage at 37°C was examined. The experiment was carried out in the same manner as in (5-1) above (Figure 10). The results demonstrated the following. FGFC and FGF2 initially exhibited almost the same level of activity. However, under temperature conditions of approximately body temperature (37°C), the remaining activity of FGF2 was decreased to approximately one-thirtieth in 24 hours after the initiation of the experiment. In contrast, in the case of FGFC, the activity was little influenced by the 24-hour storage and FGFC had activity 10 times or more higher than that of FGF2.

Accordingly, the results of (5-1) and (5-2) demonstrate that FGFC has higher stability than FGF1 and FGF2, and further that, when such FGFC is used as a substitute for the conventional FGF2 medicinal composition requiring chilled storage, it exhibits high stability even under temperature conditions ranging from room temperature to body temperature, and thus FGFC is highly advantageous as a medicinal composition.

### [Example 6]

### Stability of solution concentration of chimeric protein during storage in vessel

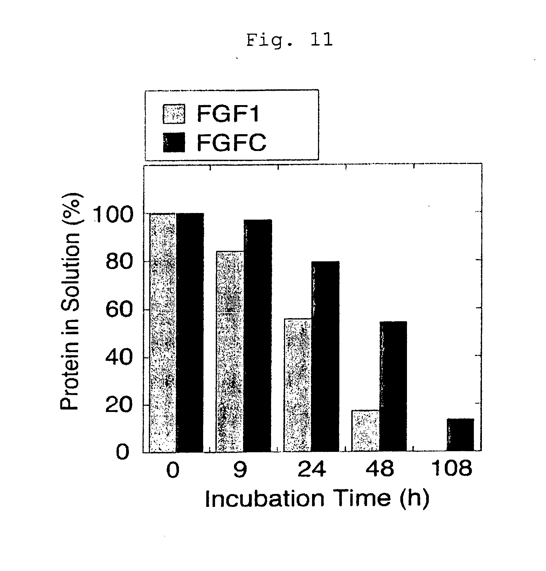

In order to examine how much of FGFC- and FGF1-containng solutions would be lost due to adsorption on storage vessel during storage at 37°C, the stability of their concentrations in solution during storage in a vessel was analyzed. 50 µl of PBS solution containing 5 µg of each FGF was placed in each well on a 96-well microtiter plate for ELISA, and it was then incubated at 37°C for various periods of time, so as to allow the protein to adsorb on the vessel. Thereafter, the whole amount of sample solution contained in the wells was recovered and then separated by SDS-polyacrylamide electrophoresis. After completion of the electrophoresis, the gel was treated with a CBB staining solution that would stain protein in proportion to its amount thereof. Thereafter, optical scanning was carried out to quantify the amount of the FGF protein remaining in the solution without being adsorbed (Figure 11).

In general, reasons for a gradual decrease in the concentration of FGF1 or FGF2 in solution with the lapse of time would be that FGF1 and FGF2 are highly adsorbed on polypropylene (a common material for sample vessels) or polystyrene (a common material for culture vessels) and that they form an aggregate in the solution to become insoluble and then precipitated. The results of the experiment shown in Figure 11 demonstrate that the concentration of FGF1 is decreased to nearly a half in 24 hours after the initiation of the experiment, whereas FGFC maintains a concentration of half or more even after 48 hours and that part of FGFC, concentration still remains even after the passage of 108 hours. This means that in the case of FGFC, a decrease of its concentration in a medicinal preparation during storage is small, and thus, FGFC is highly advantageous when it is formulated into a preparation.

### [Example 7]

### Structural stability of chimeric protein in solution (at room temperature)

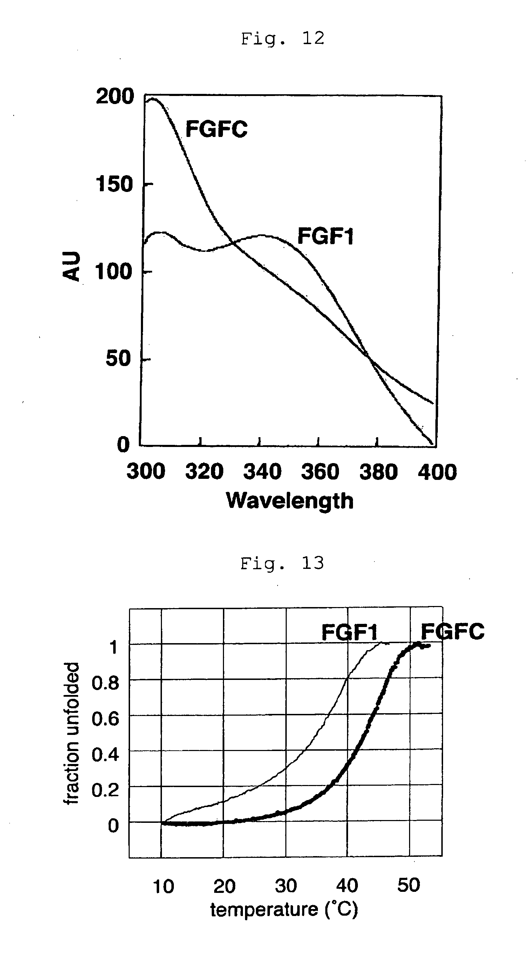

(7-1) The structural stability of each of FGFC- and FGF1-containing solutions at room temperature (25°C) was examined under the same conditions as those described in Example 6. FGF1 or FGFC prepared to a final concentration of 2 µM in a 0.7 M guanidium hydrochloride-25 mM phosphate buffer (pH 7.3) was placed in a quartz cuvette. With the temperature maintained at 25°C, both samples were excited with ultraviolet rays of 280 nm and the generated autofluorescence was examined (Figure 12). It is already known with this measurement that when the FGF protein is denatured to have its three-dimensional structure destroyed, characteristic autofluorescence intensity due to a tryptophan residue is increased around 353 nm.
   As shown in Figure 12, FGF1 exhibits strong fluorescence at around 353 nm at room temperature (25°C), and this demonstrates that the protein has a structure in which the correct folding has been partially destroyed. On the other hand, almost no such fluorescence was observed from FGFC. This demonstrates that almost all molecules are correctly folded. Accordingly, it is found that, in comparison with FGF1, FGFC has high structural stability at room temperature. Since this shows the high stability of FGFC under room-temperature conditions, it is highly advantageous when seen as a medicinal preparation.
(7-2) The structural stability of each of FGFC- and FGF1-containing solutions was examined under the same conditions as those described in (7-1) above, except that the temperature of the solution was varied. FGFC or FGF1 prepared to a final concentration of 2 µM in a 0.7 M guanidium hydrochloride-25 mM phosphate buffer (pH 7.3) was placed in a quartz cuvette. With the temperature changed from 10°C to 50°C, both samples were excited with ultraviolet rays at 280 nm and the generated autofluorescence at 353 nm was examined. The folding at 10°C was considered to be correct and the maximum value of autofluorescence at 353 nm that increased as a result of temperature rise was considered to indicate the destruction of all foldings. Based on such definitions, the proportion of the destruction of foldings (fraction unfolded) was indicated (Figure 13). As a result, in the case of FGF1, approximately 50% of molecules became unfolded at 35°C whereas the fraction unfolded of FGFC was approximately 10% around 35°C. Further, in the case of FGF1, approximately 80% of molecules became unfolded at 40°C whereas the fraction unfolded of FGFC was only approximately 30% at around 40°C. From these results, it is found that correctly folded molecules are less likely to be unfolded in FGFC than in FGF1, which means FGFC has higher structural stability in solution. Thus, FGFC is highly advantageous when seen as a medicinal preparation.

### [Example 8]

### Activity of chimeric protein to promote the proliferation of epidermal cells which is an important step in wound healing (without the addition of heparin)

(8-1) The proliferation of epidermal cells is an important step in wound healing. To date, no FGF medicaments have been placed on the market as agents that can directly promote such proliferation of epidermal cells. In the present example, the activity of promoting MK2 cells as epidermal keratinocytes was measured, and in terms of such activity, a comparison of FGFC, FGF1 and FGF2 was made.

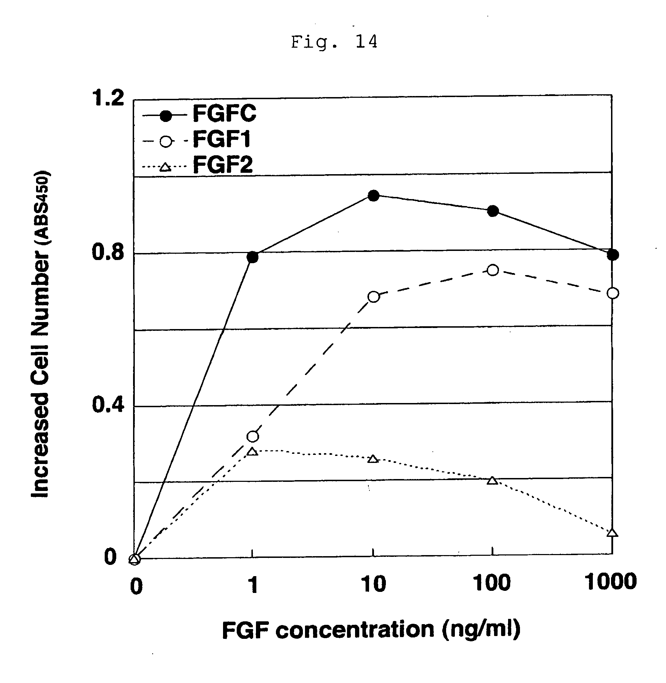

The MK2 cells were dispersed on a multi-well plate (48 wells) for tissue culture at a rate of 1 × 10⁴ cells/0.5 ml/well. The cells were cultured in a low calcium DMEM medium supplemented with 10% fetal bovine serum and EGF in a 5% CO₂ atmosphere at 37°C overnight. Thereafter, the medium was exchanged with a low calcium DMEM medium (0.25 ml/well) supplemented with 0.1% FBS. Twenty-four hours later, a chimeric protein or other samples were added in various concentrations to the culture solution. The cells were then cultured for 46 hours, and thereafter, a TetraColorOne reagent was added to the culture solution. The cells were further cultured for 4 hours. After completion of the culture, the absorbance at 450 nm of the culture solution was measured and the activity of mitochondrial enzyme that was almost proportional to the cell number was measured. The obtained value was used as an indicator of the cell number. Each point in the figure indicates the mean value of duplicate samples (Figure 14).

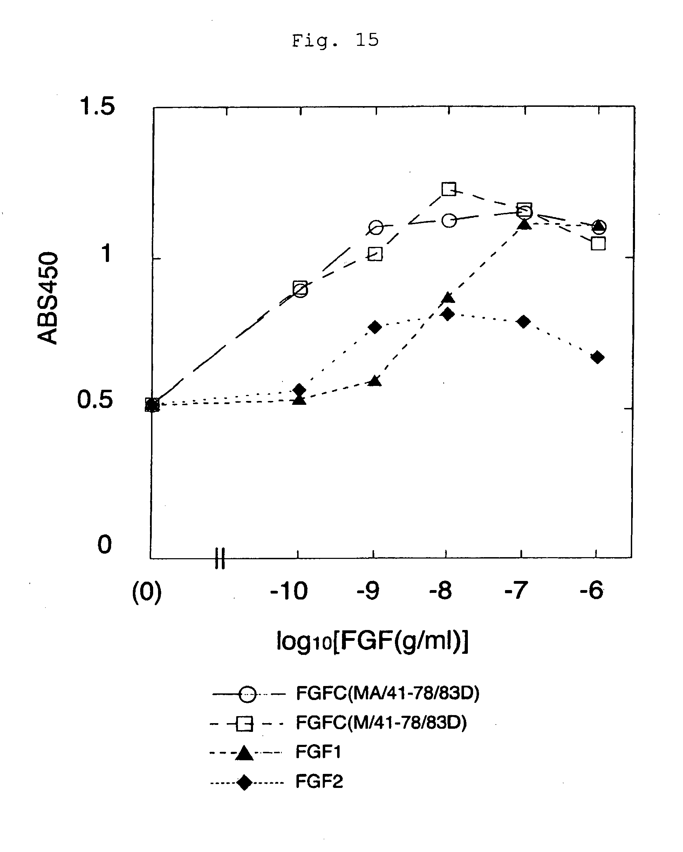

As shown in Figure 14, FGFC strongly promoted cell proliferation at a concentration of 1 ng/ml, whereas FGF1 and FGF2 promoted cell proliferation only at a level of a half or less. In addition, it was demonstrated that, at the concentrations at which the respective samples exhibited the highest activity, FGFC promoted cell proliferation more strongly than FGF1 did, and that the cell number increase attained by FGFC was larger than that attained by FGF1. FGF2 exhibited an activity even lower than FGF1. These results demonstrate that FGFC exhibited cell proliferation promoting activity that was not only higher than FGF2 abundant in epidermal cells and having no FGFR2b stimulating activity but also higher than FGF1. This demonstrates that FGFC is useful as an excellent wound healing medicinal composition.
(8-2) In the present example, the activity of promoting the proliferation of MK2 cells as epidermal keratinocytes was measured in the same manner as that described in Example (8-1) above, and in terms of such activity, a comparison of FGFC(MA/41-78/83D), FGFC(M/41-78/83D), FGF1 and FGF2 was made (Figure 15). As a result, it was confirmed that both FGFC(MA/41-78/83D) and FGFC(M/41-78/83D) strongly promoted cell proliferation, whereas FGF1 and FGF2 had weak cell proliferation promoting activity, and in particular, the activity of FGF2 was significantly low. These results demonstrate that both FGFC(MA/41-78/83D) and FGFC(M/41-78/83D) are useful as excellent wound healing medicinal compositions.

### [Example 9]

### Fibroblast proliferation promoting activity of chimeric proteins (without addition of heparin)

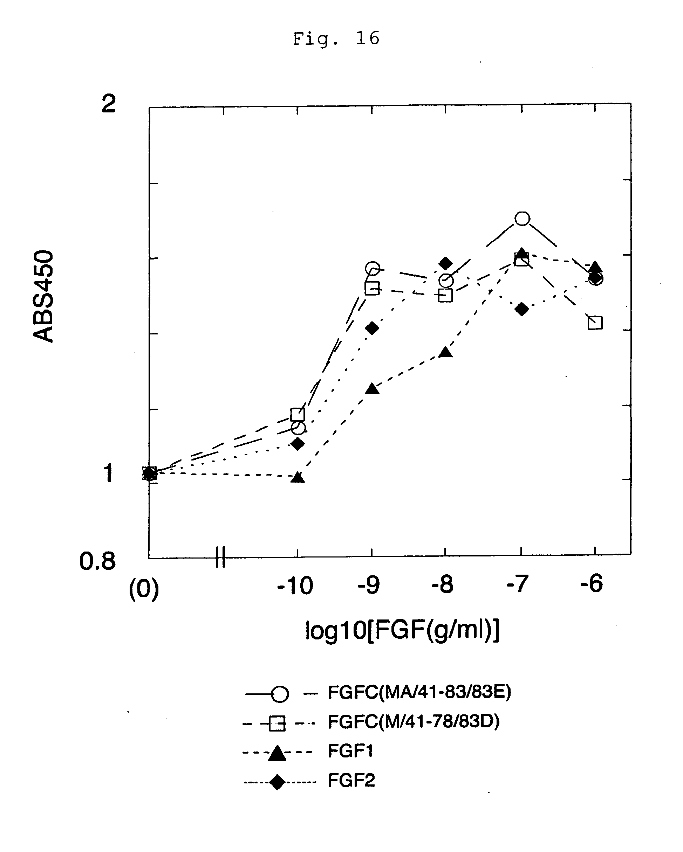

In the present experiment, the fibroblast proliferation promoting activity of each chimeric protein was measured, and in terms of such activity, a comparison of FGFC(MA/41-78/83D), FGFC(M/41-83/83E), FGF1 and FGF2 was made. Fibroblasts have the ability to synthesize a heparan sulfate sugar chain endogenously. In addition, such fibroblasts may become the target cells of FGF in vivo. Accordingly, in this experiment, it was considered that the fibroblast proliferation stimulating activity of FGF administered to a living body could be presumably evaluated based on the activity measured without the addition of heparin. Each point indicates the mean value of duplicate samples (Figure 16).

From the results shown in Figure 16, it was found that FGFC(MA/41-78/83D) and FGFC(M/41-83/83E) have strong fibroblast proliferation promoting activity without depending on exogenous heparin. It was demonstrated that such activity was equal to or somewhat higher than that of FGF2. In contrast, it was demonstrated that FGF1 had low activity under such conditions that no heparin was added. This suggests that FGF1 would need exogenous heparin for stabilizing its structure. These results demonstrate that FGFC(MA/41-78/83D) and FGFC(M/41-83/83E) are excellent wound healing medicinal compositions.

### [Example 10]

### Activity of chimeric proteins to promote prevention and treatment of radiation-induced damage to living bodies

In the present example, for the purpose of measuring the activity of chimeric proteins to promote the prevention and treatment of radiation-induced damage to living bodies, the damage on small intestine epithelial cells of a mouse caused by whole-body exposure to radiation rays and the effects of FGFC on the treatment of the damege were examined.

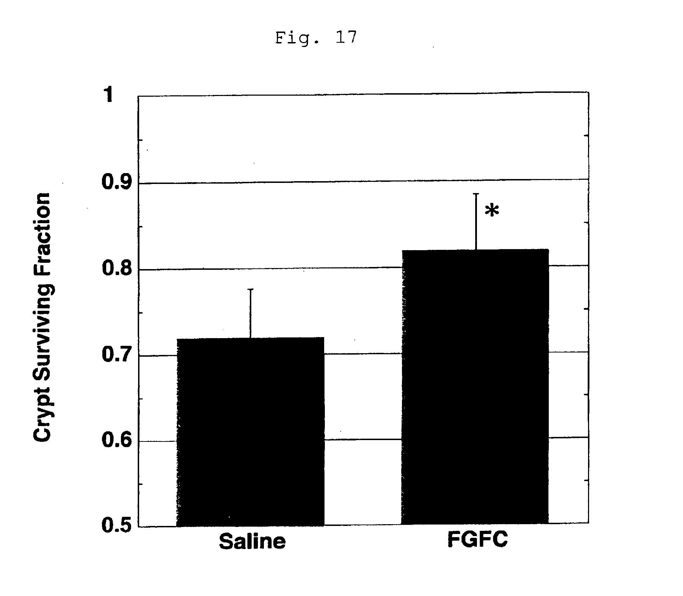

All experiments using mice were carried out with humane care in accordance with a pre-approved experimental animal plan. FGFC (10 µg) was diluted with a buffer consisting of 5 µg/ml heparin in physiological saline to a final amount of 0.5 ml. The thus prepared FGFC solution was administered into the abdominal cavity of each C3H mouse 24 hours before application of radiation rays. Gamma rays generated from ¹³⁷Cs radiation source were systemically applied to the mouse at a level of 10 gray. The dose rate was approximately 0.57 Gy/min. The mice surviving 3.5 days after the application of the radiation rays were subjected to euthanasia. A tissue sample was collected from each mouse, it was fixed with 10% formaldehyde and embedded in paraffin. It was then treated to prepare a sample for use in histological analyses. The jejunum was divided into 10 portions, and a section was then prepared from each of such 10 portions. The section was observed under a microscope and the crypt structure was scored. A crypt containing 10 or more cells was determined to be "surviving." The number of crypts was counted in 3 mice from each group, and the mean number was compared with that of an unirradiated mouse group (Figure 17).

As a result, it was observed that the FGFC administered mouse group had a significantly higher crypt surving fraction than the physiological saline administered mouse group. These results demonstrate that FGFC has excellent effects on the prevention and treatment of radiation-induced damage to the crypt. In this experimental system, the activity of FGF1 known to have the effects of preventing and treating the radiation-induced damage to the crypt was also measured and compared with the activity of FGFC. As a result, it was demonstrated that FGFC exhibited stronger activity than FGF1.

### [Example 11]

### Efficient mass-production of soluble chimeric proteins using Escherichia coli expression system

*Escherichia coli* BL21(DE3)pLysS strains transformed with pET-3c expression vectors having FGFC(MA/41-83/83K), FGFC(MA/41-83/83E), FGFC(MA/41-78/83D) and FGFC(M/41-78/83D) as inserts were cultured at the same scale according to an ordinary method, so that the *Escherichia coli* was allowed to express the respective proteins. Thereafter, the cell mass was disrupted and centrifuged, and a protein was then obtained from a soluble fraction. A protein derived from the same scale of culture solution was loaded on SDS-polyacrylamide gel, and it was then analyzed by electrophoresis (SDS-PAGE). The results are shown in Figure 18. Lane 1 indicates an unpurified cell mass-derived soluble total protein fraction. Lane 4 indicates an FGF protein in the cell mass-derived soluble total protein fraction. The FGF protein was bound to a heparin affinity chromatographic column and then eluted with highly concentrated common salt. Lane 2 indicates a pass-through fraction from each of the aforementioned columns. Lane 3 indicates a washed fraction. After completion of the electrophoresis, the gel (protein) was stained with Coomassie brilliant blue (CBB) according to an ordinary method. The arrow indicates the position of the FGFC.

It was demonstrated that both FGFC(MA/41-78/83D) and FGFC(M/41-78/83D) produce soluble proteins in larger amounts than FGFC(MA/41-83/83K) and FGFC(MA/41-83/83E). It is generally considered that FGF1 and FGF2 molecules having heparin binding ability have active-type molecule folding. Accordingly, these results suggest that FGFC(MA/41-78/83D) and FGFC(M/41-78/83D) are also produced in large amounts as their active-type molecules. Moreover, from these results, it is found that FGFC(MA/41-78/83D) and FGFC(M/41-78/83D) can produce larger amounts of soluble proteins than FGFC(MA/41-83/83K). This demonstrates that FGFC(MA/41-83/83K) formed by simply changing the amino acid (E) at position 83 of FGFC(MA/41-83/83E) to the original amino acid (K) of FGF2 does not cause an increase in the production amount.

### [Example 12]

### Wound healing promoting activity (influence on treatment of wound on mouse skin involving deficiency of all layers)

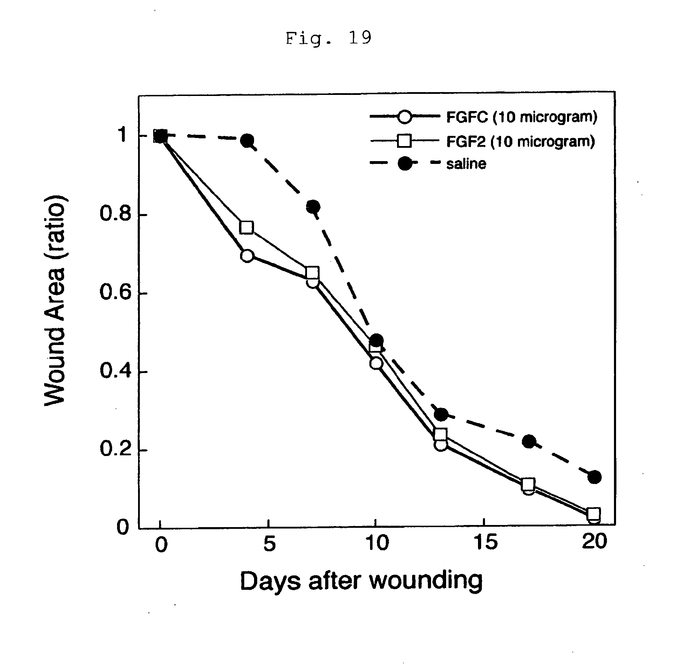

In the present example, the effects of FGFC and FGF2 on the treatment of a wound on mouse skin involving the deficiency of all skin layers were examined. The production of a wound on mouse skin involving the deficiency of all skin layers and the evaluation of the effects of FGF were carried out as follows. A diabetes model mouse db/db was anesthetized, and a round wound with an area of approximately 3 cm² which involved the deficiency of all skin layers was prepared on the dorsal portion of the mouse by surgical procedures. Thereafter, 100 µl of physiological saline containing 10 µg of FGFC or FGF2 was administered dropwise to the wound. On the other hand, physiological saline was administered dropwise to a control group. Thereafter, each mouse was kept in a clean cage. The size of the wound was measured over time, and the area was calculated. A decrease in the thus obtained wound area was compared over time, so that the activity of FGF to promote wound healing was evaluated. As a result, it was demonstrated that the area of the wound of the mice administered with FGFC and FGF2 was decreased more rapidly than that of the mice administered with physiological saline (Figure 19). It was also observed that the effects of FGFC to promote wound healing were almost equal to those of FGF2, and that, in particular, such effects of FGFC were somewhat higher than those of FGF2 for the first 5 days. That is to say, it was demonstrated that FGFC has the activity of strongly promoting wound healing that is equal to or higher than that of FGF2.

### [Example 13]

### Stem cell proliferating activity

In the present example, nerve stem cells are used to examine the effects of FGFC, FGF1 and FGF2 on such stem cells. A suspension containing nerve stem cells obtained by treating fresh central nervous tissues with enzyme is subjected to a floating culture in a serum-free medium that contains an epidermal growth factor (EGF), and FGFC, FGF1 or FGF2, and in some cases, a leukemia inhibitory factor (LIF) but which does not contain fetal bovine serum (FBS). As a result, nerve stem cells proliferate in the form of a spherical cell mass (neurosphere). The cells in the neurosphere are disintegrated, so that a subculture can be carried out. The number of the thus obtained nerve stem cells is counted to demonstrate that FGFC has an excellent stem cell proliferating activity.

Thereafter, cells that constituted the neurosphere are adhered to a coated culture dish. The growth factor is eliminated and various types of differentiation inducing factors such as fetal bovine serum are added, whereupon those cells are induced to differentiate into such cells as nerve cells, astrocytes, and oligodendrocytes. Accordingly, it can be proved that FGFC promotes the proliferation of nerve stem cells as neurosphere constituting cells.

The types of stem cells whose proliferation can be promoted by FGFC are not limited to nerve stem cells. There can be used many types of stem cells such as mesenchymal stem cells, embryonic stem cells, and induced pluripotent stem cells (iPS cells).

### Industrial Applicability

The medicinal composition of the present invention can be used as a substitute for a medicinal composition comprising FGF2 as an active ingredient and it is easier to formulate into a preparation. The present medicinal composition can be used as an agent for promoting wound healing, a medicinal composition for preventing and treating radiation-induced damage to the intestinal epithelia and the bone marrow, and in an *in vitro* method for promoting the proliferation of stem cells.

## Claims

1. An FGF2 substitute-containing medicinal composition, which comprises, as an active ingredient, an FGF1/FGF2 chimeric protein, wherein the amino acid sequence constituting the chimeric protein is shown in any one of SEQ ID NOS: 5 to 8, for use in the promotion of wound healing accompanied by the promotion of the epithelial cell proliferation.

2. An FGF2 substitute-containing medicinal composition, which comprises, as an active ingredient, an FGF1/FGF2 chimeric protein, wherein the amino acid sequence constituting the chimeric protein is shown in any one of SEQ ID NOS: 5 to 8 for use in preventing and/or treating radiation-induced damage to the intestinal canal.

3. The FGF2 substitute-containing medicinal composition for use according to claim 2, which is for use in the prevention or treatment of:
(a) intestinal inflammation generated as side effect of radiotherapy for cancer; or
(b) serious disorder of the intestinal canal in victims of nuclear accident;
wherein the canal epithelial cells have been damaged by exposure to radiation rays.

4. An FGF2 substitute-containing medicinal composition which comprises, as an active ingredient, an FGF1/FGF2 chimeric protein, wherein the amino acid sequence constituting the chimeric protein is shown in any one of SEQ ID NOS: 5 to 8 for use in preventing and/or treating radiation-induced damage to the bone marrow.

5. An *in vitro* method of promoting the proliferation of stem cells, said method comprising contacting said stem cells with an FGF2 substitute-containing medicinal composition which comprises, as an active ingredient, an FGF1/FGF2 chimeric protein, wherein the amino acid sequence constituting the chimeric protein is shown in any one of SEQ ID NOS: 5 to 8.

6. The FGF2 substitute-containing medicinal composition for use according to any one of claims 1 to 4, wherein
the chimeric protein is an active form of a chimeric protein obtained by culturing *Escherichia coli* transformed with DNA encoding the amino acid sequence shown in any one of SEQ ID NOS: 5 to 8, disrupting the cultured cells, and directly performing isolation and purification on a soluble fraction of the disrupted culture product.

7. The FGF2 substitute-containing medicinal composition for use according to any one of claims 1 to 4 or 6, which is a medicinal composition exhibiting a higher pharmacological action than a medicinal composition comprising the FGF2 protein as an active ingredient.

8. *The in vitro* method according to claim 5 wherein
the chimeric protein is an active form of a chimeric protein obtained by culturing *Escherichia coli* transformed with DNA encoding the amino acid sequence shown in any one of SEQ ID NOS: 5 to 8, disrupting the cultured cells, and directly performing isolation and purification on a soluble fraction of the disrupted culture product.

9. The *in vitro* method according to claim 5 or 8 wherein said medicinal composition exhibits a higher pharmacological action than a medicinal composition comprising the FGF2 protein as an active ingredient.

## Patentansprüche

1. FGF2-Ersatz-enthaltende medizinische Zusammensetzung, die als ein aktives Ingrediens ein chimäres FGF1/FGF2-Protein umfasst, wobei die Aminosäuresequenz, die das chimäre Protein bildet, in einer von SEQ ID NOS:5 bis 8 gezeigt ist, zur Verwendung bei der Begünstigung der Wundheilung, verbunden mit der Begünstigung der Epithelzellproliferation.

2. FGF2-Ersatz-enthaltende medizinische Zusammensetzung, die als aktives Ingrediens ein chimäres FGF1/FGF2-Protein umfasst, wobei die Aminosäuresequenz, die das chimäre Protein bildet, in einer von SEQ ID NOS:5 bis 8 gezeigt ist, zur Verwendung bei der Prävention und/oder Behandlung strahleninduzierter Schädigung am Intestinalkanal.

3. FGF2-Ersatz-enthaltende medizinische Zusammensetzung zur Verwendung gemäß Anspruch 2, die zur Verwendung in der Prävention oder Behandlung von:
(a) intestinaler Entzündung, erzeugt als Nebenwirkung einer Strahlentherapie für Krebs, oder
(b) einer ernsten Störung des Intestinalkanals bei Opfern eines Nuklearunfalls;
wobei die Kanalepithelzellen durch Exposition gegenüber Strahlung geschädigt wurden, ist.

4. FGF2-Ersatz-enthaltende medizinische Zusammensetzung, die als aktives Ingrediens ein chimäres FGF1/FGF2-Protein umfasst, wobei die Aminosäuresequenz, die das chimäre Protein bildet, in einer der SEQ ID NOS:5 bis 8 gezeigt ist, zur Verwendung bei der Prävention und/oder Behandlung einer strahlungsinduzierten Schädigung am Knochenmark.

5. In-vitro-Verfahren zur Förderung der Proliferation von Stammzellen, wobei das Verfahren Inkontaktbringen der Stammzellen mit einer FGF2-Ersatz-enthaltenden medizinischen Zusammensetzung umfasst, welche als aktives Ingrediens ein chimäres FGF1/FGF2-Protein umfasst, wobei die Aminosäuresequenz, die das chimäre Protein bildet, in einer der SEQ ID NOS:5 bis 8 gezeigt ist.

6. FGF2-Ersatz-enthaltende medizinische Zusammensetzung zur Verwendung gemäß einem der Ansprüche 1 bis 4, wobei das chimäre Protein in einer aktiven Form eines chimären Proteins ist, die erhalten wird durch Kultivieren von *Escherichia coli,* die mit DNA transformiert ist, welche die Aminosäuresequenz, die in einer der SEQ ID NOS:5 bis 8 gezeigt ist, codiert, Aufbrechen der kultivierten Zellen und direktes Durchführen einer Isolierung und Reinigung an einer löslichen Fraktion des aufgebrochenen Kulturproduktes.

7. FGF2-Ersatz-enthaltende medizinische Zusammensetzung zur Verwendung gemäß einem der Ansprüche 1 bis 4 oder 6, die eine medizinische Zusammensetzung ist, die eine höhere pharmakologische Wirkung aufweist als eine medizinische Zusammensetzung, die das FGF2-Protein als aktives Ingrediens umfasst.

8. In-vitro-Verfahren gemäß Anspruch 5, wobei das chimäre Protein eine aktive Form eines chimären Proteins ist, die durch Kultivieren von *Escherichia coli,* die mit DNA transformiert ist, welche die in einer der SEQ ID NOS:5 bis 8 gezeigte Aminosäuresequenz codiert, Aufbrechen der kultivierten Zellen und direktes Durchführen von Isolierung und Reinigung an einer löslichen Fraktion des aufgebrochenen Kulturproduktes erhalten wird.

9. In-vitro-Verfahren gemäß Anspruch 5 oder 8, wobei die medizinische Zusammensetzung eine höhere pharmakologische Wirkung als eine medizinische Zusammensetzung, die das FGF2-Protein als aktives Ingrediens umfasst, aufweist.

## Revendications

1. Composition médicinale contenant un substitut du facteur FGF2, laquelle comprend, en qualité d'ingrédient actif, une protéine chimérique FGF1/FGF2, étant entendu que la séquence d'acides aminés constituant cette protéine chimérique est l'une de celles qui sont présentées en tant que Séquences N° 5 à N° 8, pour utilisation afin de promouvoir la cicatrisation et de promouvoir aussi la prolifération de cellules épithéliales.

2. Composition médicinale contenant un substitut du facteur FGF2, laquelle comprend, en qualité d'ingrédient actif, une protéine chimérique FGF1/FGF2, étant entendu que la séquence d'acides aminés constituant cette protéine chimérique est l'une de celles qui sont présentées en tant que Séquences N° 5 à N° 8, pour utilisation afin de prévenir et/ou traiter des dommages provoqués par des radiations au niveau du tube intestinal.

3. Composition médicinale contenant un substitut du facteur FGF2 pour utilisation conforme à la revendication 1 ou 2, qui est conçue pour être utilisée dans la prévention ou le traitement
a) d'une inflammation intestinale causée, en tant qu'effet secondaire, par une radiothérapie d'un cancer,
b) ou d'un grave trouble du tube intestinal apparu chez des victimes d'un accident nucléaire,
dans laquelle ou lequel les cellules épithéliales du tube intestinal ont été endommagées par exposition à un rayonnement radiatif.

4. Composition médicinale contenant un substitut du facteur FGF2, laquelle comprend, en qualité d'ingrédient actif, une protéine chimérique FGF1/FGF2, étant entendu que la séquence d'acides aminés constituant cette protéine chimérique est l'une de celles qui sont présentées en tant que Séquences N° 5 à N° 8, pour utilisation afin de prévenir et/ou traiter des dommages provoqués par des radiations au niveau de la moelle osseuse.

5. Procédé *in vitro* permettant de promouvoir la prolifération de cellules souches, lequel procédé comporte le fait de mettre lesdites cellules souches en contact avec une composition médicinale contenant un substitut du facteur FGF2, laquelle comprend, en qualité d'ingrédient actif, une protéine chimérique FGF1/FGF2, étant entendu que la séquence d'acides aminés constituant cette protéine chimérique est l'une de celles qui sont présentées en tant que Séquences N° 5 à N° 8.

6. Composition médicinale contenant un substitut du facteur FGF2 pour utilisation conforme à l'une des revendications 1 à 4, dans laquelle la protéine chimérique est une forme active d'une protéine chimérique qu'on a obtenue en cultivant une *Escherichia coli* transformée avec un ADN codant l'une des séquences d'acides aminés présentées en tant que Séquences N° 5 à N° 8, en provoquant la rupture des cellules de cette culture, et en opérant isolement et purification directement sur une fraction soluble du produit de rupture de la culture.

7. Composition médicinale contenant un substitut du facteur FGF2 pour utilisation conforme à l'une des revendications 1 à 4 et 6, qui est une composition médicinale dotée d'une activité pharmacologique supérieure à celle d'une composition médicinale comprenant, en qualité d'ingrédient actif, la protéine FGF2.

8. Procédé *in vitro* conforme à la revendication 5, dans lequel la protéine chimérique est une forme active d'une protéine chimérique qu'on a obtenue en cultivant une *Escherichia coli* transformée avec un ADN codant l'une des séquences d'acides aminés présentées en tant que Séquences N° 5 à N° 8, en provoquant la rupture des cellules de cette culture, et en opérant isolement et purification directement sur une fraction soluble du produit de rupture de la culture.

9. Procédé *in vitro* conforme à la revendication 5 ou 8, dans lequel ladite composition médicinale est dotée d'une activité pharmacologique supérieure à celle d'une composition médicinale comprenant, en qualité d'ingrédient actif, la protéine FGF2.
